(19) Europäisches Patentamt
European Patent Office
Office européen des brevets

(11) EP 2 538 947 B1

# (12) EUROPEAN PATENT SPECIFICATION

(45) Date of publication and mention of the grant of the patent:
**17.08.2016 Bulletin 2016/33**

(21) Application number: **11707039.1**

(22) Date of filing: **24.02.2011**

(51) Int Cl.:
***A61K 31/785*** *(2006.01)* ***A61K 31/787*** *(2006.01)*
***A61K 45/06*** *(2006.01)* ***C08G 61/12*** *(2006.01)*
***C08G 73/02*** *(2006.01)* ***C08G 73/06*** *(2006.01)*
***C08G 73/18*** *(2006.01)*

(86) International application number:
**PCT/US2011/026102**

(87) International publication number:
**WO 2011/106545 (01.09.2011 Gazette 2011/35)**

## (54) POLYIMIDAZOLES FOR USE AS BILE ACID SEQUESTRANTS

POLYIMIDAZOLE ALS GALLENSÄUREKOMPLEXBILDNER

POLYIMIDAZOLES POUR EMPLOI EN TANT QU'AGENTS SÉQUESTRANTS D'ACIDE BILIAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.02.2010 US 307819 P**
**24.02.2010 US 307816 P**

(43) Date of publication of application:
**02.01.2013 Bulletin 2013/01**

(73) Proprietor: **Relypsa, Inc.**
**REDWOOD CITY CA 94063 (US)**

(72) Inventors:
- **LEES, Inez**
**Mountain View**
**California 94041 (US)**
- **BIYANI, Kalpesh**
**Newark**
**California 94560 (US)**
- **CONNOR, Eric**
**Los Gatos**
**California 94560 (US)**
- **HECKER, Scott**
**Del Mar**
**California 92014 (US)**
- **ZHANG, Hongmin**
**Fremont**
**California 94536 (US)**
- **COPE, Michael James**
**Berkeley**
**California 94702 (US)**
- **GOKA, Elizabeth**
**San Jose**
**California 95117 (US)**
- **LEE, Angela**
**San Jose**
**California 95124 (US)**
- **MADSEN, Deidre**
**Sunnyvale**
**California 94086 (US)**
- **SHAO, Jun**
**Fremont**
**California 94538 (US)**
- **ZHANG, Xinnan**
**Campbell, CA 95008 (US)**

(74) Representative: **Eisenführ Speiser**
**Patentanwälte Rechtsanwälte PartGmbB**
**Postfach 10 60 78**
**28060 Bremen (DE)**

(56) References cited:
**US-A- 5 900 233** **US-A- 6 111 056**
**US-A- 6 147 183**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 538 947 B1

## Description

FIELD OF THE INVENTION

The present invention generally relates to amine polymers useful to bind bile acids in the gastrointestinal tract of a patient in need of bile acid removal. These polymers and pharmaceutical compositions thereof are useful to lower cholesterol, particularly, non-high density lipoprotein (non-HDL), or more particularly, low-density lipoprotein (LDL) cholesterol, in patients in need thereof.

BACKGROUND OF THE INVENTION

Cholesterol is used by the body as a structural component of cell membranes. In addition, it is a basic building block for the production of many hormones, adrenal steroids, vitamin D and bile acids. Elevated levels of cholesterol carried in particles of low density lipoprotein cholesterol (LDL-C), or less specifically, cholesterol not carried in particles of high-density cholesterol (non HDL-C) are associated with an increased risk of coronary heart disease. A direct link between high blood cholesterol and cardiovascular disease (CVD) has been confirmed for both non-statin and statin trials, consistent with a direct relationship between LDL-C lowering and CVD reduction. These studies as well as many others have led to recommendations by health authorities for lowering elevated total cholesterol and LDL-C levels.

Bile acids are amphipathic detergents with micelle-forming properties that are synthesized in the liver from cholesterol and solubilize lipids to aid in their uptake from the gastrointestinal lumen. Common bile acids found in man include unconjugated bile acids (for example cholic acid, chenodeoxycholic acid, deoxycholic acid, lithocholic acid) and conjugated bile acids (for example taurocholic acid, glycocholic acid, glycochenodeoxycholic acid, taurochenodeoxycholic acid, glycodeoxycholic acid, taurodeoxycholic acid, glycolithocholic acid, and taurolithocholic acid). After a meal, bile acids are released by the gall bladder. At ileal pH, the bile acids are predominantly deprotonated and are in their salt form. The majority of bile acids are reabsorbed, primarily by active transport in the distal ileum, with elimination in the feces being the primary route of cholesterol excretion.

A bile acid sequestrant can bind bile acids to prevent reabsorption of the bile acids and cause more of the bile acids to be excreted in the stool. The sequestrant reduces the amount of bile acids reabsorbed by the intestine and subsequently transported to the liver. To compensate for this disruption in enterohepatic circulation and consequent reduction of the endogenous bile acid pool, hepatic cholesterol 7-alpha-hydroxylase is upregulated. This results in additional conversion of cholesterol into bile acids, thereby restoring the bile acid pool. Upregulation of cholesterol conversion to bile acids also involves a cascade of signaling that results in up-regulation of liver LDL-receptors and consequent lowering of serum LDL-C levels, amongst other effects.

Many bile acid sequestrants do not have the binding capacity or binding affinity to reduce the serum LDL-cholesterol concentration significantly without requiring the patient to take large amounts of the sequestrant. A large dose requirement reduces patient compliance and tolerance. Thus, bile acid sequestrants capable of removing a greater amount of bile salts from the gastrointestinal tract with equal or lower doses are needed.

SUMMARY OF THE INVENTION

The present invention provides an amine polymer that is effective for binding and removing bile salts from the gastrointestinal tract.

One aspect of the invention is an amine polymer comprising repeat units derived from polymerization of a crosslinking monomer and an imidazole monomer of formula 1 or a salt thereof

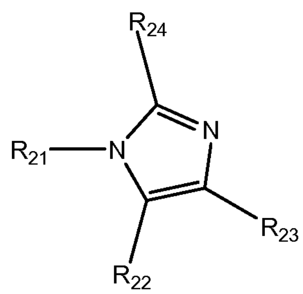

(1)

wherein $R_{21}$, $R_{22}$, $R_{23}$, and $R_{24}$ are independently hydrogen, $C_1$ to $C_{12}$ alkyl, aryl, or heterocyclo; provided that at least one of $R_{21}$, $R_{22}$, $R_{23}$, and $R_{24}$ is $-R_2$-NH-$R_{26}$ and the others are less reactive with the crosslinking monomer than the

$-R_2$-NH-$R_{26}$ nitrogen or the imidazole nitrogens; $R_2$ is $C_2$ to $C_{14}$ alkylene; $R_{26}$ is hydrogen, $C_1$ to $C_{16}$ hydrocarbyl, $C_1$ to $C_{16}$ substituted hydrocarbyl, or $C_1$ to $C_{50}$ alkyl wherein the $-CH_3$ group or one or more of the $-CH_2-$ groups are replaced with an amide, a carbonyl, an ether, an ester, a cycloalkyl, an aryl, or a heterocyclo functional group, or $C_1$ to $C_{50}$ alkyl wherein the $-CH_3$ group or one or more of the $-CH_2-$ groups are substituted with a hydroxy, a halo, an amino, an alkoxy, or an aryloxy; and the polymer segment derived from the crosslinking monomer has a calculated logP (cLog P) greater than 0.1.

Another aspect is an amine polymer comprising repeat units derived from polymerization of an amine monomer and a crosslinking monomer, the amine monomer having the structure of formula 2A

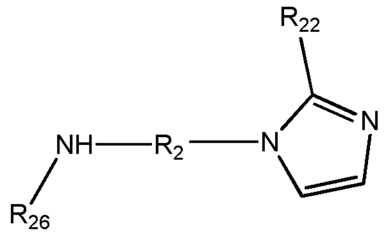

(2A)

wherein $R_{22}$ is hydrogen or $C_1$ to $C_{12}$ alkyl; $R_2$ is $C_5$ to $C_{14}$ alkylene; $R_{26}$ is hydrogen, $C_1$ to $C_{20}$ alkyl, or $C_1$ to $C_{20}$ substituted alkyl; and the crosslinking monomer is epichlorohydrin, guanidine, a guanidinium salt, a compound having the formula X-$R_1$-X, or a combination thereof, wherein each X is independently a leaving group, $R_1$ is $C_8$ to $C_{16}$ alkylene, or $C_5$ to $C_{50}$ alkylene wherein one or more of the $-CH_2-$ groups of the alkylene group is replaced with an amide, a carbonyl, an ether, an ester, a cycloalkyl, an aryl, or a heterocyclo functional group, or one or more of the $-CH_2-$ groups of the alkylene group is substituted with hydroxy.

Other objects and features will be in part apparent and in part pointed out hereinafter.

## DESCRIPTION OF THE PREFERRED EMBODIMENTS

The present invention is an amine polymer useful for binding bile salts, pharmaceutical compositions comprising the amine polymer, and methods of treating hypercholesterolemia, diabetes or other conditions that might benefit from bile acid sequestration in the gastrointestinal tract and/or increased fecal excretion of bile acids and/or bile acid metabolites, by administering the amine polymer to an animal subject in need thereof. The amine polymers exhibit increased affinity and/or capacity for binding bile salts and/or their retention as compared to commercial bile acid sequestrants. The polymers have a combination of hydrogen bonding and electrostatic properties, charged nitrogen atoms, hydrophobicity and/or polymer architecture to provide such increased affinity and/or capacity for bile salts. The terms "bile acid" and "bile salt" are used interchangeably herein and those of skill in the art will understand that a bile acid will be present in salt form and, to a lesser degree, in the protonated form in the gastrointestinal tract.

The amine polymer of the invention comprises repeat units derived from polymerization of a crosslinking monomer and an imidazole monomer of formula 1 or a salt thereof

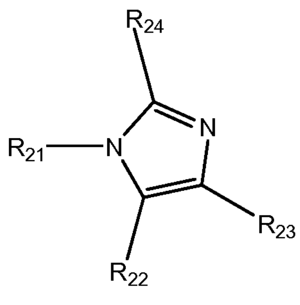

(1)

wherein $R_{21}$, $R_{22}$, $R_{23}$, and $R_{24}$ are independently hydrogen, $C_1$ to $C_{12}$ alkyl, aryl, or heterocyclo; provided that at least one of $R_{21}$, $R_{22}$, $R_{23}$, and $R_{24}$ is $-R_2$-NH-$R_{26}$ and the others are less reactive with the crosslinking monomer than the $-R_2$-NH-$R_{26}$ nitrogen or the imidazole nitrogens; $R_2$ is $C_2$ to $C_{14}$ alkylene; $R_{26}$ is hydrogen, $C_1$ to $C_{16}$ hydrocarbyl, $C_1$ to $C_{16}$ substituted hydrocarbyl, or $C_1$ to $C_{50}$ alkyl wherein one or more of the $-CH_2$-groups are replaced with an amide, a carbonyl, an ether, an ester, a cycloalkyl, an aryl, or a heterocyclo functional group, or $C_1$ to $C_{50}$ alkyl wherein one or more of the $-CH_2$-groups are substituted with a hydroxy, a halo, an amino, an alkoxy, or an aryloxy; and the polymer segment derived from the crosslinking monomer has a calculated clogP greater than 0.1.

In some embodiments, this amine polymer can comprise units derived from polymerization of an imidazole monomer having the structure of formula 2

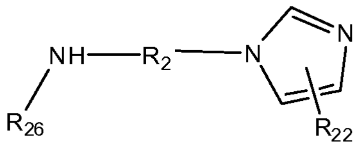

(2)

wherein $R_{22}$ is hydrogen, $C_1$ to $C_{12}$ alkyl, aryl, or heterocyclo; $R_2$ is $C_2$ to $C_{14}$ alkylene; $R_{26}$ is hydrogen, $C_1$ to $C_{20}$ alkyl, or $C_1$ to $C_{20}$ substituted alkyl. In various preferred embodiments, $R_{22}$ is hydrogen, methyl, ethyl, or propyl; $R_2$ is $C_3$ to $C_6$ alkylene; and $R_{26}$ is hydrogen or $C_1$ to $C_6$ amino-substituted alkyl; preferably, $R_{22}$ is hydrogen, $R_2$ is propylene, and $R_{26}$ is hydrogen.

Further, the amine polymer can comprise units derived from polymerization of an imidazole monomer having the structure of formula 2A

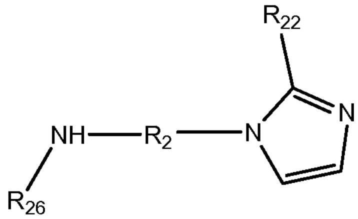

(2A)

wherein $R_{22}$ is hydrogen or $C_1$ to $C_{12}$ alkyl and $R_2$, and $R_{26}$ are defined as for formula 2.

The amine polymer described herein can contain units derived from polymerization of a crosslinking monomer that has 2 to 4 possible reactive sites and is susceptible to nucleophilic substitution. For example, the crosslinking monomer can be a dihaloalkane, a haloalkyloxirane, an alkyloxirane sulfonate, a diepoxide, a triepoxide, a tetraepoxide, a epoxyalkane, a bis(halomethyl) benzene, a tri(halomethyl) benzene, a tetra(halomethyl) benzene, a tosylate, a diglycidyl ether, a triglycidyl ether, a diglycidyl ester, a triglycidyl ester, a bis(halomethyl)aryl, or a combination thereof.

In some polymers, the crosslinking monomer is of the general formula X-$R_1$-X wherein each X is independently a leaving group and $R_1$ is $C_2$ to $C_{16}$ alkylene, arylene, -NH-C(NH)-NH-, -NH-C($NH_2^+$)-NH-, dimethylbiphenyl, or $C_2$ to $C_{50}$ alkylene wherein one or more of the -$CH_2$- groups of the alkylene group is replaced with an amide, a carbonyl, a cycloalkyl, an ether, an ester, a cycloalkyl, an aryl, or a heterocyclo functional group. In this regard, preferably, $R_1$ is $C_2$ to $C_{16}$ alkylene, $C_2$ to $C_{16}$ arylene or $C_2$ to $C_{50}$ alkylene wherein one or more of the-$CH_2$- groups of the alkylene group is replaced with an amide, a carbonyl, a cycloalkyl, an ether, an ester, a cycloalkyl, an aryl, or a heterocyclo functional group. In some of the embodiments, $R_1$ is $C_2$ to $C_{16}$ alkylene; preferably, $R_1$ is $C_8$ to $C_{12}$ alkylene.

In some of the embodiments, X is halo, epoxy, diaziridino, mesylate, sulfate, phosphate, aldehyde, ketone, or a combination thereof. Leaving groups are well known and can be selected from those known in the art, such as those in Larock, Comprehensive Organic Transformations (VCH 1989), e.g., p. 397 et seq.

In particular, the crosslinking monomer is 1,2- dibromoethane, 1,3-dichloropropane, 1,2-dichloroethane, 1-bromo-2-chloroethane, 1,3-dichloropropane, 1,3-dibromopropane, 1,4-dichlorobutane), 1,4-dibromobutane, 1,5-dichloropentane, 1,5-dibromopentane, 1,6-dichlorohexane, 1,6-dibromohexane, 1,7-dichloroheptane, 1,7-dibromoheptane, 1,8-dichlorooctane, 1,8-dibromooctane, 1,9-dichlorononane, 1,9-dibromononane, 1,10-dichlorodecane, 1,10-dibromodecane, 1,11-dibromoundecane, 1,11-dichloroundecane, 1,12-dichlorododecane, 1,12-dibromododecane, iodomethyl)oxirane, 1,3-butadiene diepoxide, 1,5-hexadiene diepoxide, 4-tosyloxy-1,2-epoxybutane, bromo-1,2-epoxybutane, 1,2,7,8-diepoxyoctane, 1,4-bis(2',3'-epoxypropyl)perfluoro-n-butane, 1,2,9,10-diepoxydecane, tris(2,3-epoxypropyl) isocyanurate), 1,4-dichloromethylbenzene, 1,4-dibromomethylbenzene, 1,2,3-trichlorobenzene, 1,2,4-trichlorobenzene, 1,3,5-trichlorobenzene, 1,2,3-tribromobenzene, 1,2,4-tribromobenzene, 1,3,5-tribromobenzene, 1,2,4,5-tetrachlorobenzene, 1,2,4,5-tetrabromobenzene, glycidyl tosylate, glycidyl 3-nitrobenzenesulfonate, glycidyl methacrylate, diglycidyl ether, ethylene glycol diglycidyl ether, propylene glycol diglycidyl ether, 1 ,4-butanediol diglycidyl ether, 1,2-ethanedioldiglycidyl ether, glycerol diglycidyl ether, 1,3-diglycidyl glyceryl ether, neopentyl glycol diglycidyl ether, diethylene glycol diglycidyl ether, 1,4- bis(glycidyloxy)benzene, resorcinol digylcidyl ether, 1,6-hexanediol diglycidyl ether, trimethylolpropane diglycidyl ether, 1,4-cyclohexanedimethanol diglycidyl ether, 2,2'-bis(glycidyloxy)diphenylmethane, bis[4-(glycidyloxy)phenyl]methane, bisphenol A diglycidyl ether, 1,3-bis(3-glycidoxypropyl)tetramethyldisiloxane, 9,9-bis[4-(glycidyloxy)phenyl]fluorine, 4,4'-methylenebis(N,N- diglycidylaniline), N,N-diglycidylaniline), triglycidyl isocyanurate, glycerol

triglycidyl ether, N,N- diglycidyl-4-glycidyloxyaniline, trimethylolethane triglycidyl ether, trimethylolpropane triglycidyl ether, glycerol propoxylate triglycidyl ether, triphenylolmethane triglycidyl ether), 1,2-cyclohexanedicarboxylic acid diglycidyl ester, isocyanuric acid (S,S,S)-triglycidyl ester, isocyanuric acid (R,R,R)-triglycidyl ester, 1,3-bis-(2,3-epoxypropyloxy)-2-(2,3-dihydroxypropyloxy)propane, 2, 6-di(oxiran-2-ylmethyl)-1, 2,3,5,6,7- hexahydropyrrolo[3,4-f]isoindol-1,3,5,7-tetraone, ethyl 5- hydroxy-6,8-di(oxiran-2-ylmethyl)-4-oxo-4h-chromene-2-carboxylate, bis[4-(2,3-epoxy- propylthio)phenyl]-sulfide, triepoxyisocyanurate, 3,7,14- tris[[3-(epoxypropoxy)propyl]dimethylsilyloxy]-1,3,5,7,9,11,14-heptacyclopentyltricyclo [7.3.3.15,11] heptasiloxane, bis(halomethyl)benzene, bis(halomethyl)biphenyl, bis(halomethyl)naphthalene, bis(2-chloroethyl)ammonium chloride, tris(2-chloroethyl)ammonium chloride, methyl chloroacetate, or a combination thereof.

More particularly, the crosslinking monomer is 1,2-dibromoethane, 1,3-dichloropropane, 1,2-dichloroethane, 1-bromo-2-chloroethane, 1,3-dichloropropane, 1,3-dibromopropane, 1,4-dichlorobutane), 1,4-dibromobutane, 1,5-dichloropentane, 1,5-dibromopentane, 1,6-dichlorohexane, 1,6-dibromohexane, 1,7-dichloroheptane, 1,7-dibromoheptane, 1,8-dichlorooctane, 1,8-dibromooctane, 1,9-dichlorononane, 1,9-dibromononane, 1,10-dichlorodecane, 1,10-dibromodecane, 1,11-dibromoundecane, 1,11-dichloroundecane, 1,12-dichlorododecane, 1,12-dibromododecane, or a combination thereof. Even more particularly, the crosslinking monomer is 1,8-dichlorooctane, 1,8-dibromooctane, 1,9-dichlorononane, 1,9-dibromononane, 1,10-dichlorodecane, 1,10-dibromodecane, 1,12-dichlorododecane, 1,11-dibromoundecane, 1,11-dichloroundecane, 1,12-dibromododecane, or a combination thereof.

For the amine polymers described herein, the molar ratio of the imidazole monomer to the crosslinking monomer is from about 1:1 to about 1:5 when the crosslinking monomer is difunctional. When the crosslinking monomer is trifunctional, the molar ratio of the imidazole monomer to the crosslinking monomer is from about 2:1 to about 1:5. Preferably, the molar ratio of the imidazole monomer to the crosslinking monomer is from about 1:1 to about 1:2.

It should be noted that the polymers of this invention are generally crosslinked. In this regard, at least some nitrogen atoms are linked by segments to other nitrogen atoms to link the polymer chains together, with the polymer ultimately taking a hydrogel and/or bead form.

Many of the amine polymers described herein can undergo a post polymerization reaction, which comprises reaction of the amine polymer with at least one additional crosslinking monomer or a ligand. When the amine polymers undergo such a post polymerization reaction with two crosslinking monomers, the reaction can proceed with both the crosslinking monomers present (e.g., by using cross linking monomers with different reactivity rates) or the amine monomer can react with one crosslinking monomer and then react with the second crosslinking monomer (e.g., the cross linking monomers are added sequentially to the reactor or the polymer is recovered prior to reaction with the second cross linking monomer). These reactions with two or more different crosslinking monomers can provide improved yield or improved physical characteristics. When these further reactions occur with an additional ligand, the crosslinking monomer and the ligand can be added simultaneously or sequentially as well.

Without wishing to be bound by any particular theory, the invention herein uses a combination of positive charge density and hydrophobicity to achieve unexpected bile acid binding affinity, binding capacity, retention and removal. The charge density comes from a concentration of positively charged nitrogen atoms that are separated by from the hydrophobic segment by one to six atoms. Hydrophobicity is expressed by the calculated log P, as discussed herein.

In some of the amine polymers, the calculated log P (cLog P) is greater than 0.1, 0.5, 1, 1.5, 2, 2.5, 3, 3.5, 4, 4.5, 5, 5.5, 6, 6.5, or 7. The Calculated log P (cLog P) (Clog P) is determined by drawing the structure of the crosslinker without the leaving groups in Chemdraw Ultra 11.0 (CambridgeSoft, Cambridge MA) and replacing the leaving groups with hydrogen, and selecting the chemical properties tool to calculate the Clog P. For example, for the crosslinker 1,10-dibromodecane, one would enter the structure of decane into Chemdraw and select "show chemical properties" from the "view" toolbar to calculate its Clog P as 5.984. If the crosslinker is a ring structure that opens during crosslinking, such as epichlorohydrin, the Clog P is determined by drawing the ring-opened structure as shown below for epichlorohydrin:

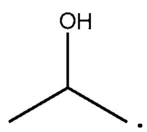

For example, the calculated log P (cLog P) for various segments is detailed in Table 1.

Table 1. Calculated log P(cLog P) of selected segments

| Segment | Calculated log P |
|---|---|
| 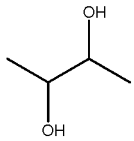 | -0.7512 |
| 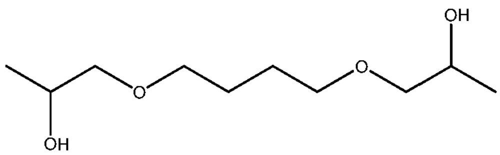 | -0.5108 |
| 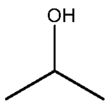 | 0.0740 |
| 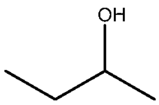 | 0.603 |
| 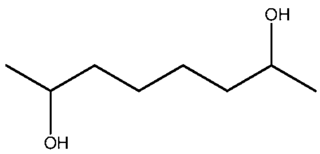 | 0.512 |
|  | 1.752 |
| 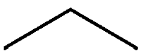 | 2.28 |
| 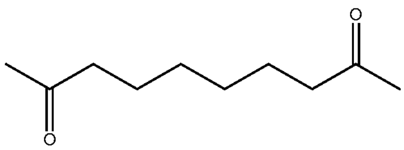 | 1.006 |
| 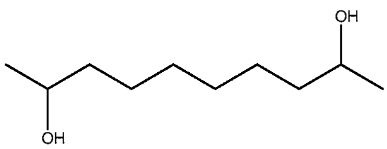 | 1.57 |
| 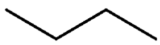 | 2.81 |
| 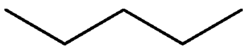 | 3.339 |
| 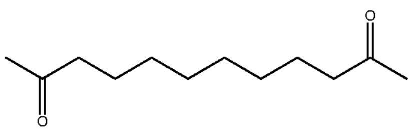 | 2.064 |
| 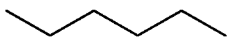 | 3.868 |
| 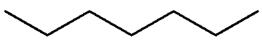 | 4.397 |

(continued)

| Segment | Calculated log P |
|---|---|
| 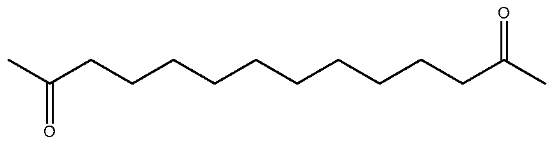 | 3.122 |
| 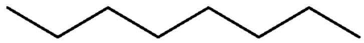 | 4.926 |
| 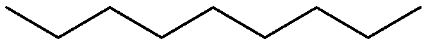 | 5.455 |
| 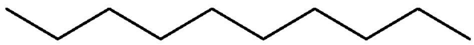 | 4.67 |
| 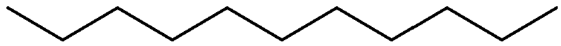 | 5.984 |
| 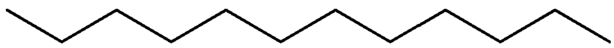 | 7.042 |
| 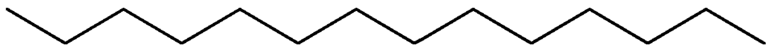 | 8.1 |
| 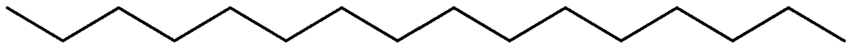 | 9.158 |

Also, in many embodiments, the amine polymer contains a permanent positive charge localized on a nitrogen atom. Without being bound by theory, it is believed that these centers of permanent positive charge separated by hydrophobic segments provide an improved material to bind bile acids. It is further believed that this combination of permanently positively charged regions and hydrophobic regions are similar to the structure of bile acids, providing better affinity as well as a better ability to bind and retain the bile acids using multiple attractive forces.

Also, the amine polymer can comprise repeat units derived from polymerization of the crosslinking monomer and an imidazole monomer, or a salt thereof, and the amine polymer has a binding capacity for bile acids of at least 2 mmol/gram of polymer when measured using an *in vitro* B assay or the amine polymer has a binding affinity for bile acids of at least 0.40 mmol/g when measured using an *in vitro* A assay.

The amine polymers or pharmaceutical compositions thereof are preferably free-flowing powders.

In order to prepare various amine polymers of the invention, the imidazole monomer is contacted with a crosslinking monomer. Depending on the particular imidazole monomer and crosslinking monomer, a solvent can be used. The solvent can be N,N-dimethyl formamide (DMF), N,N-dimethylacetamide (DMAC), tetrahydrofuran (THF), methyltetrahydrofuran, dimethylsulfoxide (DMSO), 1,4-dioxane, 2-pyrrolidinone, methylpyrrolidone (NMP), methanol, ethanol, isopropyl alcohol, butanol, pentanol, ethylene glycol or water. Mixtures of solvents can also be used. A base can be added to the reaction mixture depending on the particular imidazole monomer and crosslinking monomer. The base can be sodium carbonate, potassium carbonate, cesium carbonate, ammonium hydroxide and sodium hydroxide, triethylamine, trioctylamine, 1,4-diazabicyclo[2.2.2]octane (DABCO), 1,8-diazabicyclo[5.4.0]undec-7-ene (DBU) or N,N-diisopropylmethylamine. After the imidazole monomer and crosslinking monomer are contacted, the reaction mixture is heated to from about 40°C to about 80°C or about 70°C for about 12 to 24 hours. After the reaction is complete, the polymer product is washed with an aqueous acidic solution, then lyophilized until dry.

The amine polymers of the invention have various chemical, structural and physical properties that contribute to their capacity for binding bile acids and/or their affinity for binding bile acids preferentially over fatty acids, phosphates and/or other compounds present in the gastrointestinal tract.

The amine polymer can be administered in the form of a salt, or as a partial salt, or as salt free base. The "salt" has nitrogen atoms or groups in all or some of the repeat units that are protonated to create a positively charged nitrogen atom associated with a negatively charged counterion. The anionic counterions can be selected to minimize adverse effects on the patient. Examples of suitable counterions include $Cl^-$, $Br^-$, $CH_3OSO_3^-$, $HSO_4^-$, $SO_4^{2-}$, nitrate, $HCO_3^-$, $CO_3^{2-}$, acetate, lactate, phosphate, hydrophosphate, fumarate, malate, pyruvate, malonate, benzoate, glucuronate, oxalate, acetylglycinate, succinate, propionate, butyrate, ascorbate, citrate, tartrate, maleate, folate, an amino acid derivative, a nucleotide, a lipid, a phospholipid, or a combination thereof. The counterions can be the same as, or different from, each other. For example, the reaction product can contain two different types of counterions. In most cases, not all of the

nitrogen atoms will be in a salt form, with the percent of nitrogen atoms in a salt form being dictated by certain properties, such as flowability, storage time, and weight.

To determine the *in vitro* binding affinity for bile salts under conditions that are intended to mimic in certain respects those conditions found in the lower small intestine, the amine polymer is analyzed using assay A. The A assay combines the polymer to be analyzed in a desired concentration with a solution that mimics certain conditions present in the lower small intestine as described in Protocol 1 in the examples. After a period of time, the polymers are recovered by centrifugation and the supernatants are sampled, filtered to remove any remaining particulates and assayed for ion concentrations by liquid chromatography (LC). By comparing the equilibrium concentrations of glycocholate ($GC_{eq}$), glycodeoxycholate ($GDC_{eq}$), oleyl glycerol ($OG_{eq}$) and/or oleic acid ($OA_{eq}$) in the presence of the polymer to their concentrations in test solution in the absence of the polymer, the amount of each component bound under these experimental conditions in mmoles/g polymer is calculated. The *in vitro* bile salt binding affinity under the conditions of the A assay in Protocol 1 results in a maximum of about 0.75 mmol/gram polymer. Thus, the *in vitro* bile salt binding affinity for the amine polymers of this invention is from about 0.3 to about 0.75 mmol/gram polymer, particularly from about 0.46 to about 0.75 mmol/gram polymer, and more particularly, from about 0.55 to about 0.75 mmol/gram polymer when measured in the Assay A solution. In some cases the concentration of phosphate ions was also determined on a strong anion exchange column by liquid chromatography using a basic mobile phase in order to measure the phosphate binding affinity. The polymers of the invention bind phosphate *in vitro* in an amount of less than 0.2 mmol/gram of polymer, particularly up to about 0.15 mmol/gram polymer, and more particularly, up to about 0.10 mmol/gram polymer when measured using an A assay.

To determine the *in vitro* binding capacity for bile salts under conditions that are intended to mimic in certain respects those conditions found in the upper small intestine after a meal, the amine polymer is analyzed using Assay B. In Assay B, the polymer to be analyzed is combined in a desired concentration with a solution that mimics certain conditions present in the upper small intestine as described in Protocol 2 in the examples. The same general procedure as described above was used to calculate the amount of each component bound. The *in vitro* bile salt binding capacity under the conditions of the B assay in Protocol 2 results in a maximum of about 3.7 mmol/gram polymer. Thus, the *in vitro* bile salt binding capacity for the amine polymers is from about 0.95 to about 3.7 mmol/gram polymer, particularly from about 1.8 to about 3.7 mmol/gram polymer, and more particularly from about 2.46 to about 3.7 mmol/gram polymer when measured in the assay B solution.

To determine the *in vivo* binding retention for bile salts, the amine polymer is analyzed in a hamster model. The hamster model provides a complex and relevant measure of the polymer's binding capacity for bile acids, its binding affinity for bile acids over other anions, and its ability to retain bound bile acids and to increase the excretion of bile acids and bile acids metabolites from the gastrointestinal tract into the feces. Preferably, Golden Syrian hamsters may be used as they have a similar bile acid profile to that of humans. Male Golden Syrian hamsters are acclimated and then placed on a high-fat, high-sucrose western diet, D12079B (Research Diet, New Brunswick, NJ) for several days before the study is started. The amine polymers to be analyzed are blended into western diet at the desired dose to prepare the test diets. The hamsters are held in individual metabolic cages allowing the separation and collection of feces. Animals from the test groups are switched to the test diets, while animals from the untreated group are kept on western diet without added amine polymer. Food intake is measured for four consecutive days. For each hamster, feces from the last three days of the treatment period are collected, pooled, lyophilized, and then homogenized by grinding in a mortar and pestle. The feces samples are then extracted for fecal bile salt analysis. In some cases, a baseline treatment period is conducted where all groups of animals are placed in metabolic cages as described above and fed only on western diet without added test article. Feces are collected as described above and the effect of the amine polymer on bile salt fecal excretion is determined by comparing baseline versus treatment periods. Otherwise, the effect of amine polymers on bile salt fecal excretion is determined by comparing untreated versus test groups. Hamster fecal bile salts are analyzed as described in the examples. The amine polymers can have a calculated *in vivo* binding capacity at least 25%, 50%, 75%, 100%, 125%, 150%, 175% or 200% greater than colesevelam hydrochloride when measured at a dosage of 0.5% of the total feed intake in male Golden Syrian hamsters fed a Western diet.

The amine polymers can have a calculated *in vivo* bile salt binding capacity of at least about 0.35 mmol bile salt/gram of polymer when measured in humans. The amine polymers can have an *in vivo* binding capacity in a human of at least 0.35 mmol bile salt per gram of polymer, at least 0.4 mmol bile salt per gram of polymer, at least 0.5 mmol bile salt per gram of polymer, at least 0.6 mmol bile salt per gram of polymer, or more.

Polymers of the invention are crosslinked materials, meaning that they do not generally dissolve in solvents however they can swell with solvents or absorb the solvent. As used herein, "swelling ratio" refers to the number of grams of solvent taken up by one gram of crosslinked polymer when equilibrated in an aqueous environment. The swelling ratio is sensitive to the polymer solvent interaction parameter as described in Flory Huggins (Flory P. J. "Principles of Polymer Chemistry, Cornell Ithica Pub. 1953). When more than one measurement of swelling is taken for a given polymer, the mean of the measurements is taken to be the swelling ratio. The swelling ratio in water, or in physiological isotonic buffer, which is representative of the gastrointestinal tract (for example United States Pharmacopeia Simulated

Intestinal Fluid or Simulated Gastric Fluid), is typically in the range of about 1 to about 10 g of swelling solution (solvent)/g of polymer, particularly about 2 to 6, and more particularly about 2 to about 4. The counterion content of the polymer can affect the swelling ratio, in the examples listed below, a chloride counterion is used, and the chloride content is stated. The counterion content can be as much as 25 wt% of the total weight of the polymer and as little as <1% of the total weight of the polymer.

The amine polymers can be particles having a mean diameter from about 10 microns to about 200 microns. In some of the embodiments, the amine polymer particles are substantially spherical beads. These beads can have a mean diameter from about 10 microns to about 200 microns. As used herein, the term "substantially" means generally rounded particles having an average aspect ratio of about 1.0 to about 2.0. Aspect ratio is the ratio of the largest linear dimension of a particle to the smallest linear dimension of the particle. Aspect ratios may be easily determined by those of ordinary skill in the art. This definition includes spherical particles, which by definition have an aspect ratio of 1.0. In some embodiments, the particles have an average aspect ratio of about 1.0, 1.2, 1.4, 1.6, 1.8 or 2.0. The particles may be round or elliptical when observed at a magnification wherein the field of view is at least twice the diameter of the particle.

The substantially spherical beads can be prepared using methods known to a person skilled in the art. For example, a preferred mode of synthesis is a heterogeneous process. Such processes are also referred to as polymerization in dispersed media and include direct or inverse suspension, emulsion, precipitation, dispersion or micro emulsion polymerization, reaction in aerosol or using bulk polymerization methods. In inverse suspension, the continuous phase can be selected from apolar solvents such as silicone, toluene, benzene, hydrocarbon solvents or oils, halogenated solvents, supercritical carbon dioxide, and the like. The discrete phase for the inverse suspension system comprises solubilizing the monomer and crosslinker in water; this can be achieved by the addition of an acid such as hydrochloric acid to form the amine salt, which renders the organic amine substantially more water soluble and dispersing the amine solution in a water-immiscible solvent to form an emulsion. With a direct suspension or emulsion process, water can be used as the continuous phase, although salt brines are also useful to "salt out" the monomer and crosslinker into the discrete phase, as described in US Patent 5,414,068. The monomers can be dispersed either neat or as a solution in the continuous phase using a cosolvent. The crosslinking monomer can be added to the reaction in a semicontinuous fashion (staged addition) allowing the polymerization reaction to occur. Isolation of the beads can be carried out by filtration, washing and drying. Size can be further controlled or modified by reduction processes such as extrusion and grinding.

Polymers can be obtained by methods known to those in the art, examples of which are illustrated in the Examples herein. The crosslinked amine polymer particle is generally a reaction product of a reaction mixture that is subjected to reaction conditions. The reaction mixture may also generally contain components that are not chemically incorporated into the product. The reaction mixture typically comprises monomers.

In general, the reactions are conducted such that a polymer network is generated, which is insoluble but can be solvated into a gel. When the interpenetrating solvent is water, the insoluble material is described as a hydrogel. The reaction is carried either in solution, in bulk (i.e. using the neat monomers and crosslinking compounds) or in dispersed media. The reaction may start with the introduction of for example, temperature change or irradiation. In general amine polymers can be prepared by chain growth or step growth. Step growth polymerization involves the polymerization of monomers that contain unsaturated functional groups, including radical polymerization, cationic polymerization and anionic polymerization. Step growth polymerization involves the reaction of bifunctional or polyfunctional monomers that grow via, dimers, trimers to longer oligomers. Network formation occurs when the polymer chains react with each other. Parameters that effect the network formation reaction include temperature, solvent choice, the concentrations of monomers and crosslinkers, and the ratio of the monomer to the crosslinking monomer. The addition of a base maybe desired in some cases.

Polymerization reactions to prepare the amine polymers include preparing an aqueous solution of the amine monomer, optionally with a surfactant, and adding an organic phase containing an organic solvent and optionally, a surfactant, to the aqueous phase. The crosslinker then can be added in a batch or a semi-continuous fashion. For example, the crosslinker can be added to the polymerization all at once or can be added slowly over a period of time.

The amine polymer particles have a mean diameter of from about 10 μm to about 200 μm. Specific ranges are where the amine polymer particles have a mean diameter of from about 20 μm to about 200 μm, from about 20 μm to about 150 μm, or from about 20 μm to about 125 μm. Other ranges include from about 35 μm to about 150 μm, from about 35 μm to about 125 μm, from about 50 μm to about 125 μm, or from about 50 μm to about 100 μm. Particle sizes, including mean diameters, distributions, etc. can be determined using techniques known to those of skill in the art. For example, U.S. Pharmacopeia (USP) <429> discloses methods for determining particle sizes.

Various amine polymer particles also have less than about 4 volume percent of the particles that have a diameter of less than about 10 μm; particularly, less than about 2 volume percent of the particles that have a diameter of less than about 10 μm; more particularly, less than about 1 volume percent of the particles that have a diameter of less than about 10 μm; and even more particularly, less than about 0.5 volume percent of the particles that have a diameter of less than about 10 μm. In other cases, specific ranges are less than about 4 volume percent of the particles that have

a diameter of less than about 20 μm; less than about 2 volume percent of the particles that have a diameter of less than about 20 μm; less than about 1 volume percent of the particles that have a diameter of less than about 20 μm; less than about 0.5 volume percent of the particles that have a diameter of less than about 20 μm; less than about 2 volume percent of the particles that have a diameter of less than about 30 μm; less than about 1 volume percent of the particles that have a diameter of less than about 30 μm; less than about 1 volume percent of the particles that have a diameter of less than about 30 μm; less than about 1 volume percent of the particles that have a diameter of less than about 40 μm; or less than about 0.5 volume percent of the particles that have a diameter of less than about 40 μm. In various embodiments, the amine polymer has a particle size distribution wherein not more than about 5 volume% of the particles have a diameter less than about 30 μm (i.e., D(0.05) < 30 μm), not more than about 5 volume% of the particles have a diameter greater than about 250 μm (i.e., D(0.05) > 250 μm), and at least about 50 volume% of the particles have a diameter in the range from about 70 to about 150 μm.

The particle distribution of the amine polymer can be described as the span. The span of the particle distribution is defined as (D(0.9)-D(0.1))/D(0.5), where D(0.9) is the value wherein 90% of the particles have a diameter below that value, D(0.1) is the value wherein 10% of the particles have a diameter below that value, and D(0.5) is the value wherein 50% of the particles have a diameter above that value and 50% of the particles have a diameter below that value as measured by laser diffraction. The span of the particle distribution is typically from about 0.5 to about 1, from about 0.5 to about 0.95, from about 0.5 to about 0.90, or from about 0.5 to about 0.85. Particle size distributions can be measured using Niro Method No. A 8 d (revised September 2005), available from GEA Niro, Denmark, using the Malvern Mastersizer.

It has now been found that when using the amine polymers and the compositions of the present invention, a once-a-day dose is substantially equivalent to a twice-a-day dose, which is also substantially equivalent to a three-times-a-day dose. Generally, the once per day or twice per day administration of a daily amount of the polymer or the composition has a bile acid removal that is not statistically significantly different from the removal of the same polymer or composition at the same daily amount administered three times per day.

Additionally, the invention is directed to methods of removing bile acids from an animal subject by administering an amine polymer or a pharmaceutical composition comprising an amine polymer, wherein less than 25% of subjects taking the polymer or composition once per day experience mild or moderate gastrointestinal adverse events at a dose of 6.0 grams/day or less. Gastrointestinal adverse events may include flatulence, diarrhea, abdominal pain, constipation, stomatitis, nausea and/or vomiting. In some aspects, the polymer or composition is administered twice a day and less than 25% of subjects taking the polymer or composition twice per day experience mild or moderate gastrointestinal adverse events. In some instances, the subjects taking the polymer or composition once per day or twice per day experience no severe gastrointestinal adverse events. The amine polymers or pharmaceutical compositions of the present invention have about 50% or more tolerability as compared to the same polymer or composition of the same daily amount administered three times a day. For example, for every two patients in which administration of the polymer three times a day is well tolerated, there is at least one patient in which administration of the polymer once a day or twice a day is well tolerated.

When administration is well tolerated, there should be little or no significant dose modification or dose discontinuation by the subject. In some embodiments, well tolerated means there is no apparent dose response relationship for gastrointestinal adverse events. In some of these embodiments, well tolerated means that the following gastrointestinal adverse effects are not reported from a statistically significant number of subjects, including those effects selected from the group consisting of flatulence, diarrhea, abdominal pain, constipation, stomatitis, nausea and vomiting.

In other embodiments, the present invention provides a method of removing bile acids from the gastrointestinal tract of an animal subject in need thereof, comprising administering an effective amount of an amine polymer or a composition comprising an amine polymer, wherein the polymer or composition is as well tolerated as administering substantially the same amount of the same polymer or composition three times per day. In some instances, the subject is experiencing hypercholesteremia and thus the method treats hypercholesteremia. In other instances, the method lowers serum cholesterol.

Without wanting to be bound by any particular theory, the tolerability of the polymer or composition comprising the polymers results from physical properties that the amine polymers may possess, including a viscosity when hydrated and sedimented of from about 10,000 Pa·s to about 2,500,000 Pa·s, from about 10,000 Pa·s to about 2,000,000 Pa·s, from about 10,000 Pa·s to about 1,500,000 Pa·s, from about 10,000 Pa·s to about 1,000,000 Pa·s, from about 10,000 Pa·s to about 500,000 Pa·s, or from about 10,000 Pa·s to about 250,000 Pa·s, from about 30,000 Pa·s to about 3,000,000 Pa·s, from about 30,000 Pa·s to about 2,000,000 Pa·s, or from about 30,000 Pa·s to about 1,000,000 Pa·s, the viscosity being measured at a shear rate of 0.01 $sec^{-1}$. This viscosity is measured using a wet polymer prepared by mixing the polymer thoroughly with a slight excess of simulated intestinal fluid (per USP <26>), allowing the mixture to sediment for 3 days at 37°C, and decanting free liquid from the sedimented wet polymer. The steady state shear viscosity of this wet polymer can be determined using a Bohlin VOR Rheometer (available from Malvern Instruments Ltd., Malvern, U.K.) or equivalent with a parallel plate geometry (upper plate of 15 mm diameter and lower plate of 30 mm diameter, and gap between plates of 1 mm) and the temperature maintained at 37°C.

The amine polymers may further have a hydrated and sedimented yield stress of from about 150 Pa to about 4000 Pa, from about 150 Pa to about 3000 Pa, from about 150 Pa to about 2500 Pa, from about 150 Pa to about 1500 Pa, from about 150 Pa to about 1000 Pa, from about 150 Pa to about 750 Pa, or from about 150 Pa to about 500 Pa, from about 200 Pa to about 4000 Pa, from about 200 Pa to about 2500 Pa, from about 200 Pa to about 1000 Pa, or from about 200 Pa to about 750 Pa. Dynamic stress sweep measurements (i.e., yield stress) can be made using a Reologica STRESSTECH Rheometer (available from Reologica Instruments AB, Lund, Sweden) or equivalent in a manner known to those of skill in the art. This rheometer also has a parallel plate geometry (upper plate of 15 mm diameter, lower plate of 30 mm diameter, and gap between plates of 1 mm) and the temperature is maintained at 37°C. A constant frequency of 1 Hz with two integration periods can be used while the shear stress is increased from 1 to $10^4$ Pa.

Amine polymers used in this invention may also have desirable compressibility and bulk density when in the form of a dry powder. Some of the particles of the amine polymers in the dry form have a bulk density of from about 0.8 $g/cm^3$ to about 1.5 $g/cm^3$, from about 0.82 $g/cm^3$ to about 1.5 $g/cm^3$, from about 0.84 $g/cm^3$ to about 1.5 $g/cm^3$, from about 0.86 $g/cm^3$ to about 1.5 $g/cm^3$, from about 0.8 $g/cm^3$ to about 1.2 $g/cm^3$, or from about 0.86 $g/cm^3$ to about 1.2 $g/cm^3$. The bulk density affects the volume of amine polymer needed for administration to a patient. For example, a higher bulk density means that a lower volume will provide the same number of grams of amine polymer. This lower volume can improve patient compliance by allowing the patient to perceive they are taking a smaller amount due to the smaller volume.

A powder composed of the particles of the amine polymer in dry form has a compressibility index of from about 3 to about 30, from about 3 to about 25, from about 3 to about 20, from about 3 to about 15, from about 3 to about 13, from about 5 to about 25, from about 5 to about 20, or from about 5 to about 15. The compressibility index is defined as 100*(TD-BD)/TD, wherein BD and TD are the bulk density and tap density, respectively. Bulk density (BD) and tapped density (TD) are used to calculate a compressibility index (CI). Standardized procedures for this measurement are specified as USP <616>. A quantity of the powder is weighed into a graduated cylinder. The mass M and initial (loosely packed) volume Vo are recorded. The cylinder is then placed on an apparatus which raises and then drops the cylinder, from a height of 3 mm ±10%, at a rate of 250 times (taps) per minute. The volume is measured after 500 taps and then again after an additional 750 taps (1250 total). If the difference in volumes after 500 and 1250 taps is less than 2%, then the final volume is recorded as Vf and the experiment is complete. Otherwise, tapping is repeated in increments of 1250 taps at a time, until the volume change before and after tapping is less than 2%. The following quantities are calculated from the data:

$$\text{Bulk Density (BD)} = M/Vo$$

$$\text{Tapped Density (TD)} = M/Vf$$

$$\text{Compressibility Index (CI, also called Carr's Index)} = 100*(TD-BD)/TD$$

The powder form of the amine polymers settles into its smallest volume more easily than polymers conventionally used to treat hypercholesteremia. This makes the difference between the bulk density and the tap density (measured powder density after tapping a set number of times) from about 3% to about 30%, from about 3% to about 25%, from about 3% to about 20%, from about 3% to about 15%, from about 3% to about 10%, from about 5% to about 35%, from about 5% to about 30%, or from about 5% to about 20% of the bulk density.

The polymers and pharmaceutical compositions described herein retain a significant amount of the bound bile salts throughout the small intestine, and specifically, the bile salts bound by the polymer are not released prior to entry into the colon or excretion of the polymer in the feces. The term "significant amount" as used herein is not intended to mean that the entire amount of the bound bile salt are retained prior to fecal excretion or entry in to the colon. A sufficient amount of the bound bile salts are retained, such that a therapeutic and/or prophylactic benefit is obtained. For example, it may be sufficient for a polymer to retain bile acids such that there is a significant increase in the amount of bile acids entering the colon. The bile acids may then be released from the polymer but may still substantially be excreted either intact or as metabolites in the feces and thus for purposes of this invention have been sufficiently retained. Retention of bile acids may be measured by measuring the amounts of bile acids in the feces or in colonic aspirates or extracts above baseline levels (i.e., above the amount of bile acids retained in the feces when no polymer is administered to the animal subject). Particular amounts of bound bile salts that can be retained range from about 5% to about 100% above baseline levels. The polymer or pharmaceutical composition should retain at least about 5% of the bound bile salts, more particularly at least about 10%, even more particularly at least about 25% and most particularly at least about 50% of the bound

bile salts above baseline levels. Retention of bile acids by the polymer can be calculated either directly by *in vitro* methods or indirectly by *in vivo* methods. The period of retention is generally during the time that the polymer or composition is being used therapeutically or prophylactically. When the polymer or composition is used to bind and remove bile salts from the gastrointestinal tract, the retention period is the time of residence of the polymer or composition in the gastrointestinal or the average residence time of the polymer or composition in the small intestine.

The polymers and pharmaceutical compositions described herein may result in an increased ratio of primary to secondary bile acids excreted in the feces. Bile acids may be characterized by their site of synthesis and modification; primary bile acids (for example cholic acid and chenodeoxycholic acid) are synthesized in hepatocytes from cholesterol and secondary or tertiary bile acids (for example deoxycholic acid and lithocholic acid) are the products of bacterial dehydroxylation in the terminal ileum and colon. Primary bile acids may be deconjugated and/or dehydroxylated to convert them to secondary or tertiary bile acids; for example deoxycholate (from cholate) and lithocholate (from chenodeoxycholate). A change in the ratio of excreted bile acids towards primary or unmetabolized bile acids is a measure of *in vivo* retention of bile acids by polymers. The amine polymers, in an *in vivo* measurement, can produce on average at least 11%, 15%, 20%, 30%, or more primary bile acids in the feces based on total bile acids in the feces.

Generally, the amine polymers are not significantly absorbed from the gastrointestinal tract. Depending upon the size distribution of the amine polymer particles, clinically insignificant amounts of the polymers may be absorbed. More specifically, about 90% or more of the polymer is not absorbed, about 95% or more is not absorbed, even more specifically about 97% or more is not absorbed, and most specifically about 98% or more of the polymer is not absorbed.

The amine polymers can be used to remove bile salts from an animal subject by administering an effective amount of the polymer to an animal subject in need thereof. The bile salts can be bound and retained by the amine polymer and then removed from the gastrointestinal tract in the feces. Further, the amine polymers can be used to reduce serum LDL-cholesterol, or serum non-HDL-cholesterol, in an animal subject. In some instances, the mean serum LDL can be decreased by at least 15%, at least 20%, at least 25%, at least 30% or more after 2, 4, 12, 26, 52 or more weeks of treatment with the amine polymer at a daily dose at which the subject experiences no severe gastrointestinal adverse events. In some instances, the daily dose of the amine polymer is about 6.0 g/day, 5.0 g/day, 4.0 g/day, 3.0, 2.5, or 2.0 g/day or less.

Further, the amine polymers can be administered to improve glycemic control in a human subject with Type II diabetes mellitus. Preferably, when a human subject with Type II diabetes mellitus is treated, glycated hemoglobin ($Hb_{A1c}$) can be decreased by at least 0.5%, at least 0.6%, at least 0.7%, at least 0.8%, at least 0.9%, at least 1.0% or more after 18, 26, 52 or more weeks of treatment with the amine polymer at a daily dose at which the subject experiences no severe gastrointestinal adverse events. In some instances, the daily dose of the amine polymer is about 6.0 g/day, 5.0 g/day, 4.0 g/day, 3.0, 2.5, or 2.0 g/day or less. Also, the fasting plasma glucose can be decreased by at least 14 mg/dL (0.8 mmol/L), at least 16 mg/dL (0.9 mmol/L), at least 18 mg/dL (1 mmol/L), at least 20 mg/dL (1.1 mmol/L) or more after 2, 4, 12, 26, 52 or more weeks of treatment with the amine polymer at a daily dose at which the subject experiences no severe gastrointestinal adverse events. In some instances, the daily dose of the amine polymer is about 6.0 g/day, 5.0 g/day, 4.0 g/day, 3.0, 2.5, or 2.0 g/day or less.

Further, the amine polymers can be used to ameliorate, treat or slow progression of Alzheimer's disease.

The amine polymers can also be used to treat non-alcoholic statohepatitis, cholestatic pruritus, irritable bowel syndrome with diarrhea (IBS-D), idiopathic bile acid malabsorption, genetic or congenital Fibroblast Growth Factor 19 (FGF 19) deficiency or a combination thereof. When the amine polymers are used to treat cholestatic pruritus, they can be used in combination with an oral or topical antipruritic containing, for example, an antihistamine, a corticosteroid, a local anesthetic, a counterirritant, an opioid, an opioid receptor antagonist, or other therapies including but not limited to crotamiton, doxepin, mirtazapine, capsaicin, tacrolimus, linoleic acid, gabapentin, activated charcoal, thalidomide, naltrexone, erythropoietin, nicergoline, naltrexone, nalmefene, butorphanol, naloxone, rifampin, ondansetron, ursodeoxycholate, S-adenosyl-L-methionine, serotonin-selective reuptake inhibitors, phenobarbital, dronabinol, phototherapy, or a combination thereof.

When the amine polymers are used to treat IBS-D, they can be used in combination with antidiarrheals such as opiates, opioid or opioid analogs including loperamide, codeine, diphenoxylate, serotonin receptor antagonists such as alosetron, ramosetron and cilansetron, serotonin-selective reuptake inhibitors, tricyclic antidepressants such as amitriptyline and desipramine or drugs reducing the levels of serotonin (5-HT), antispasmodic drugs including anticholinergics such as hyoscyamine or dicyclomine, chloride secretion blockers such as crofelemer and probiotics.

As used herein, an animal subject can be a human or other mammal in need of either bile salt removal, reduction of serum LDL-cholesterol, or non HDL-cholesterol concentration, increase in HDL-C or improved glycemic control.

The methods, polymers and compositions described herein are suitable for removal of bile salts from an animal subject wherein the subject is in need of such bile salt removal. For example, patients experiencing hypercholesterolemia or hyperlipidemia benefit from such bile salt removal. The methods described herein are applicable to these patients regardless of the underlying condition that is causing the high serum cholesterol levels or need for bile acid removal.

The amine polymers can be administered once, twice, or three times a day. If the amine polymer is administered

once a day, it may be administered just before, with, or just after the largest meal of the day. Also, if administered once a day, it may be administered in connection with the largest, on average during a twenty-four hour period, release of bile acids from the gall bladder, which is typically in the morning. Further, it is preferred that the amine polymer is administered at least 3 hours before or after any agents that might have an adverse interaction with the amine polymers.

The dosage regimen to treat hypercholesterolemia, atherosclerosis, diabetes, Alzheimer's disease, non-alcoholic steatohepatits, cholestatic pruritus, IBS-D, idiopathic bile acid malabsorption or reduce plasma cholesterol with the combination therapy and pharmaceutical compositions of the present invention can be selected using a variety of factors. These include the type, age, weight, sex, diet, and medical condition of the patient, the severity of the disease, the route of administration, pharmacological consideration such as the activity, efficacy, pharmacokinetics and toxicology profiles of the particular compound employed, whether a drug delivery system is utilized, and whether the amine polymer is administered as part of a drug combination. Thus, the dosage regimen actually employed may vary widely.

Initial treatment of a patient suffering from a hyperlipidemic condition such as hypercholesterolemia and/or atherosclerosis can begin with the dosages indicated above. Treatment should generally be continued as necessary over a period of several weeks to several months or years until the condition has been controlled or eliminated. Patients undergoing treatment with the amine polymers disclosed herein can be routinely monitored by, for example, measuring serum LDL and total cholesterol levels by any of the methods well known in the art, to determine the effectiveness of the combination therapy. Repeated analysis of such data permits modification of the treatment regimen during therapy so that optimal effective amounts of each type of agent are administered at any point in time, and so that the duration of treatment can be determined as well. In this way, the treatment regimen/dosing schedule can be rationally modified over the course of therapy so that the lowest amount of amine polymer and optionally, combination treatment, is administered and so that administration is continued only so long as is necessary to successfully treat the hyperlipidemic condition such as hypercholesterolemia and atherosclerosis.

If necessary, the amine polymers or pharmaceutical compositions may be administered in combination with other therapeutic agents. The choice of therapeutic agents that can be co-administered with the compounds of the invention will depend, in part, on the condition being treated. For example, various agents can be co-administered with the amine polymer, including agents used in reducing serum LDL-cholesterol or non-HDL-cholesterol, which comprise a hydroxymethyl-glutaryl-coenzyme A (HMG CoA) reductase inhibitor, a fibrate, a cholesterol absorption inhibitor, niacin (i.e. nicotinic acid or derivatives thereof), a phytosterol, an intestinal lipase inhibitor, an intestinal or secreted phospholipase A2 inhibitor, inhibitors of the synthesis or normal activity of Apo-B100, agonists of the synthesis or normal activity of ApoA, or any agent that modulates cholesterol absorption or metabolism, or a combination thereof. In some instances, the HMG CoA reductase inhibitor comprises a statin, such as atorvastatin, cerivastatin, fluvastatin, lovastatin, mevastatin, pitavastatin, pravastatin, rosuvastatin, simvastatin, or a combination thereof. The cholesterol absorption inhibitor can comprise ezetimibe. The fibrate can be benzafibrate, ciprofibrate, clofibrate, gemfibrozil, fenofibrate, or a combination thereof. The intestinal lipase inhibitor can comprise orlisatat. In some instances, the amine polymers or pharmaceutical compositions may be administered in combination with a HMG CoA reductase inhibitor and niacin (e.g., lovastatin and niacin), or a HMG CoA reductase inhibitor and a cholesterol absorption inhibitor (e.g., simvastatin and ezetimibe), or a HMG CoA reductase inhibitor and an intestinal lipase inhibitor.

In another example, other agents can be co-administered with the amine polymer, including agents used in preventing or treating diabetes, obesity or other dyslipidemias, such as a sulfonylurea, a biguanidine, a glitazone, a thiazolidindione, an activator of peroxisome proliferator-activated receptors (PPARs), an alpha-glucosidase inhibitor, a potassium channel antagonist, an aldose reductase inhibitor, a glucagon antagonist, a retinoid X receptor (RXR) antagonist, a farnesoid X receptor (FXR) agonist, a FXR antagonist, glucagon-like peptide-1 (GLP-1), a GLP-1 analog, a dipeptidyl peptidase IV (DPP-IV) inhibitor, amylin, an amylin analog, an SGLT2 inhibitor, insulin, an insulin secretagogue, a thyroid hormone, a thyroid hormone analog, an alpha glucosidase inhibitor or a combination thereof. The biguanidine can be metformin, buformin, phenformin, or a combination thereof. The thiazolidindione can be pioglitazone, rivoglitazone, rosiglitazone, troglitazone, or a combination thereof. The sulfonylurea can be acetohexamide, chlorpropamide, tolbutamide, tolazamide, glipizide, gliclazide, glibenclamide, gliquidone, glyclopyramide, glimepiride, or a combination thereof. The DPP-IV inhibitor can be alogliptin, linagliptin, saxagliptin, sitagliptin, vildagliptin, or a combination thereof. The GLP-1 analog can be exenatide, liraglutide, albiglutide, or a combination thereof. The alpha glucosidase inhibitor can be acarbose, miglitol or voglibose.

The term dyslipidemia is taken to mean a deviation in at least one of total serum cholesterol, LDL-cholesterol, non-HDL cholesterol, HDL-cholesterol or triglyceride from that considered normal by the National Cholesterol Education Program or other suitable bodies. In another example, other agents can be co-administered with the amine polymer, including an anti-platelet agent, a beta- blocker, a renin-angiotensin-aldosterone system (RAAS) inhibitor, a RAAS modulator (e.g., angiotensin converting enzyme inhibitors, renin inhibitors, angiotensin receptor blockers, aldosterone antagonists or sodium channel blockers, including amiloride, triamterene, trimethoprim, and pentamidine) or a combination thereof.

The amine polymers can also be administered with other cholesterol-lowering agents such as acifran, azacosterol, benfluorex, β-benzalbutyramide, carnitine, chondroitin sulfate, clomestrone, detaxtran, dextran sulfate sodium, 5,8,11,14,17-eicosapentaenoic acid, eritadenine, furazabol, meglutol, melinamide, mytatrienediol, ornithine, γ-oryzanol, pantethine, pentaerythritol tetraacetate, α-phenybutyramide, priozadil, probucol, β-sitosterol, sultosilic acid, piperazine salt, tiadenol, triparanol, xenbucin, or a combination thereof.

Other agents that can be advantageously used for treatment in combination with the amine polymers are a squalene epoxidase inhibitor, a squalene synthetase inhibitor (or squalene synthase inhibitor), an acyl-coenzyme A, cholesterol acyltransferase (ACAT) inhibitor (including selective inhibitors of ACAT-1 or ACAT-2, as well as dual inhibitors of ACAT-1 and ACAT-2), a microsomal triglyceride transfer protein (MTP) inhibitor, probucol, a cholesterol absorption inhibitor (e.g., ezetimibe and 1-(4-fluorophenyl)-3(R)-3(S)-(4-fluorophenyl)-3-hydroxypropyl), 4(S)-4-hydroxyphenol (-2-azetidinone) described in U.S. Pat. Nos. 5,727,115 and 5,846,966), a LDL receptor inducer, a platelet aggregation inhibitor (e.g., a glycoprotein IIb/IIa fibrinogen receptor antagonist), aspirin, vitamin $B_6$ (or pyridoxine), vitamin $B_{12}$ (or cyanocobalamin), a water-soluble pharmaceutical salt or ester of folic acid (e.g., sodium salt and the methylglucamine salt), an anti-oxidant vitamin (e.g., vitamin C and E and beta-carotene), or a combination thereof.

The term "treating" as used herein includes achieving a therapeutic benefit. By therapeutic benefit is meant eradication, amelioration, or prevention of the underlying disorder being treated. For example, in a hypercholesterolemia patient, therapeutic benefit includes eradication or amelioration of the underlying hypercholesterolemia. Also, a therapeutic benefit is achieved with the eradication, amelioration, or prevention of one or more of the physiological symptoms associated with the underlying disorder such that an improvement is observed in the patient, notwithstanding that the patient may still be afflicted with the underlying disorder. In some treatment regimens, the amine polymer or composition of the invention may be administered to a patient at risk of developing hypercholesterolemia or diabetes or to a patient reporting one or more of the physiological symptoms of hypercholesterolemia or diabetes, even though a diagnosis of hypercholesterolemia or diabetes may not have been made.

The pharmaceutical compositions of the present invention include compositions wherein the amine polymers are present in an effective amount, i.e., in an amount effective to achieve therapeutic or prophylactic benefit. The actual amount effective for a particular application will depend on the patient (e.g., age, weight, etc.), the condition being treated, and the route of administration. Determination of an effective amount is well within the capabilities of those skilled in the art, especially in light of the disclosure herein. The effective amount for use in humans can be estimated from animal models. For example, a dose for humans can be formulated to achieve gastrointestinal concentrations that have been found to be effective in animals. In various embodiments, the human patient takes about 0.5 g to about 10 g per day, preferably about 0.5 g to about 5 g per day, more preferably, about 0.5 g to about 3 g per day, about 0.5 g to about 2.5 g per day, and most preferably about 0.5 g to about 2.0 g per day.

The polymers and compositions described herein can be used as food products and/or food additives. They can be added to foods prior to consumption or during packaging.

The amine polymers or pharmaceutically acceptable salts thereof, or compositions described herein, can be delivered to the patient using a wide variety of routes or modes of administration. The most preferred routes for administration are oral, intestinal, or rectal. Rectal routes of administration are known to those of skill in the art. Intestinal routes of administration generally refer to administration directly into a segment of the gastrointestinal tract, e.g., through a gastrointestinal tube or through a stoma. The most preferred route for administration is oral.

The polymers (or pharmaceutically acceptable salts thereof) may be administered per se or in the form of a pharmaceutical composition wherein the active compound(s) is in admixture or mixture with one or more pharmaceutically acceptable excipients. Pharmaceutical compositions for use in accordance with the present invention may be formulated in a conventional manner using one or more pharmaceutically acceptable excipients comprising carriers, diluents, and auxiliaries which facilitate processing of the active compounds into preparations which can be used physiologically. Proper composition is dependent upon the route of administration chosen.

For oral administration, the polymers or compositions of the invention can be formulated readily by combining the polymer or composition with pharmaceutically acceptable excipients well known in the art. Such excipients enable the compositions of the invention to be formulated as powders, tablets, pills, dragees, capsules, liquids, gels, syrups, slurries, suspensions, wafers, and the like, for oral ingestion by a patient to be treated. Pharmaceutical preparations for oral use can be obtained as a solid excipient, optionally grinding a resulting mixture, and processing the mixture of granules, after adding suitable auxiliaries, if desired, to obtain tablets or dragee cores. Suitable excipients are, in particular, fillers such as sugars, including lactose or sucrose; cellulose preparations such as, for example, maize starch, wheat starch, rice starch, potato starch, gelatin, gum tragacanth, methyl cellulose, hydroxypropyl methylcellulose, sodium carboxymethylcellulose, and/or polyvinyl pyrrolidone (PVP); and various flavoring agents known in the art. If desired, disintegrating agents may be added, such as the cross-linked polyvinyl pyrrolidone, agar, or alginic acid or a salt thereof such as sodium alginate.

Additionally, the amine polymer composition can comprise one or more fat-soluble vitamins such as vitamin A, D, E, K, or a combination thereof. An amount of the fat-soluble vitamin can be added to the composition sufficient to deliver about the daily dietary intake level (i.e., the Reference Daily Intake (RDI)), which is currently 3000 IU, 400 IU,

30 IU, 80 μg, respectively, for vitamin A, D, E, and K.

In various embodiments, the active ingredient (e.g., polymer) constitutes over about 20%, more particularly over about 50%, even more particularly over about 75%, and most particularly more than about 90% by weight of the oral dosage form, the remainder comprising suitable excipient(s).

The amine polymers or pharmaceutical compositions can be administered in the form of a chewable or mouth-disintegrating tablet, a liquid, a powder, a powder contained within a sachet, a soft gelatin capsule, or a hard gelatin capsule. In some embodiments, the polymers of the invention are provided as pharmaceutical compositions in the form of liquid compositions. In various embodiments, the pharmaceutical composition contains an amine polymer dispersed in a suitable liquid excipient. Suitable liquid excipients are known in the art; see, e.g., Remington's Pharmaceutical Sciences.

An effective amount of the polymers of the invention can be administered to the animal subject in less than four unit doses per day, such as in less than four tablets per day. The "dosage unit" or "unit dose" is a tablet, capsule or other oral dosage form containing an amount of the amine polymer. The polymer is generally administered in 4, 3, 2 or 1 unit doses in a 24-hour period, which provides a daily dose of the polymer to the subject under treatment.

Unless otherwise indicated, an "alkyl" group as described herein alone or as part of another group is an optionally substituted linear saturated monovalent hydrocarbon radical containing from one to twenty carbon atoms and preferably one to twelve carbon atoms, or an optionally substituted branched saturated monovalent hydrocarbon radical containing three to twenty carbon atoms, and preferably three to eight carbon atoms. Examples of unsubstituted alkyl groups include methyl, ethyl, n-propyl, i-propyl, n-butyl, i-butyl, s-butyl, t-butyl, n-pentyl, i-pentyl, s-pentyl, t-pentyl, and the like.

The term "amide" as used herein represents a bivalent (i.e., difunctional) amido linkage (i.e.,

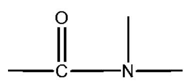

).

The term "aryl" as used herein alone or as part of another group denotes an optionally substituted monovalent aromatic hydrocarbon radical, preferably a monovalent monocyclic or bicyclic group containing from 6 to 12 carbons in the ring portion, such as phenyl, biphenyl, naphthyl, substituted phenyl, substituted biphenyl or substituted naphthyl. Phenyl and substituted phenyl are the more preferred aryl groups. The term "aryl" also includes heteroaryl.

The term "cycloalkyl" as used herein denotes optionally an optionally substituted cyclic saturated monovalent bridged or non-bridged hydrocarbon radical containing from three to eight carbon atoms in one ring and up to 20 carbon atoms in a multiple ring group. Exemplary unsubstituted cycloalkyl groups include cyclopropyl, cyclobutyl, cyclopentyl, cyclohexyl, cycloheptyl, cyclooctyl, adamantyl, norbornyl, and the like.

The term "-ene" as used as a suffix as part of another group denotes a bivalent radical in which a hydrogen atom is removed from each of two terminal carbons of the group, or if the group is cyclic, from each of two different carbon atoms in the ring. For example, alkylene denotes a bivalent alkyl group such as methylene ($-CH_2-$) or ethylene ($-CH_2CH_2-$), and arylene denotes a bivalent aryl group such as o-phenylene, m-phenylene, or p-phenylene.

The term "ether" as used herein represents a bivalent (i.e., difunctional) ether linkage (i.e., -O-).

The term "ester" as used herein represents a bivalent (i.e., difunctional) ester linkage (i.e., -C(O)O-).

The term "heteroaryl," as used herein alone or as part of another group, denotes an optionally substituted monovalent monocyclic or bicyclic aromatic radical of 5 to 10 ring atoms in protonated or unprotonated form, where one or more, preferably one, two, or three, ring atoms are heteroatoms independently selected from N, O, and S, and the remaining ring atoms are carbon. Exemplary heteroaryl moieties include benzofuranyl, benzo[d]thiazolyl, benzo[d]thiazolium, isoquinolinyl, isoquinolinium, quinolinyl, quinolinium, thiophenyl, imidazolyl, imidazolium, oxazolyl, oxazolium, furanyl, thiazolyl, thiazolium, pyridinyl, pyridinium, furyl, thienyl, pyridyl, pyrrolyl, pyrrolidinium, indolyl, indolinium, and the like.

The term "heterocyclo," as used herein alone or as part of another group, denotes a saturated or unsaturated monovalent monocyclic group of 4 to 8 ring atoms in protonated or unprotonated form, in which one or two ring atoms are heteroatom(s), independently selected from N, O, and S, and the remaining ring atoms are carbon atoms. Additionally, the heterocyclic ring may be fused to a phenyl or heteroaryl ring, provided that the entire heterocyclic ring is not completely aromatic. Exemplary heterocyclo groups include the heteroaryl groups described above, pyrrolidino, pyrrolidinium, piperidino, piperidinium, morpholino, morpholinium, piperazino, piperazinium, and the like.

The term "hydrocarbon" as used herein describes a compound or radical consisting exclusively of the elements carbon and hydrogen.

The term "substituted" as in "substituted aryl," "substituted alkyl," and the like, means that in the group in question (i.e., the alkyl, aryl or other group that follows the term), at least one hydrogen atom bound to a carbon atom

is replaced with one or more substituent groups such as hydroxy (-OH), alkylthio, phosphino, amido (-CON($R_A$)($R_B$), wherein $R_A$ and $R_B$ are independently hydrogen, alkyl, or aryl), amino(-N($R_A$)($R_B$), wherein $R_A$ and $R_B$ are independently hydrogen, alkyl, or aryl), halo (fluoro, chloro, bromo, or iodo), silyl, nitro (-$NO_2$), an ether (-$OR_A$ wherein $R_A$ is alkyl or aryl), an ester (-OC(O)$R_A$ wherein $R_A$ is alkyl or aryl), keto (-C(O)$R_A$ wherein $R_A$ is alkyl or aryl), heterocyclo, and the like. When the term "substituted" introduces a list of possible substituted groups, it is intended that the term apply to every member of that group. That is, the phrase "optionally substituted alkyl or aryl" is to be interpreted as "optionally substituted alkyl or optionally substituted aryl."

As used herein "possible reaction sites" in the amine monomers are nitrogen atoms bonded to one or more hydrogen atoms.

Having described the invention in detail, it will be apparent that modifications and variations are possible without departing from the scope of the invention defined in the appended claims.

EXAMPLES

The following non-limiting examples are provided to further illustrate the present invention. The following assays were used for the *in vitro* and *in vivo* testing detailed in the examples below.

Protocol 1: Conditions mimicking the lower small intestine (A assay). Amine polymers were measured in conditions mimicking those found in the lower small intestine (Northfield, TC and McColl, I (1973) "Postprandial concentrations of free and conjugated bile salts down the length of the normal human small intestine", Gut 14: 513-518, Borgstrom, B, et al. (1957) "Studies of intestinal digestion and absorption in the human", J Clin Invest 36: 1521-1536.)

The following test solution was prepared: 50mM N,N-Bis(2-hydroxyethyl)-2-aminoethanesulfonic acid (BES), 50mM sodium BES, 6.5mM sodium phosphate, 0.93mM sodium glycocholate, 0.93mM sodium glycodeoxycholate, 150mM sodium chloride, pH 7.0. The test solution was stored at -20°C. Before use the test solution was thawed in a 37°C water bath, stirred vigorously on a stir plate for greater than 20 minutes, and filtered through a Nalgene 0.45 micron cellulose nitrate filter unit. This was found to provide reproducible results. Amine polymers to be analyzed were freeze-dried a minimum of 18 hours and were accurately dispensed into 16x100mm borosilicate test tubes, with each tube containing between 23 and 28 mg of test sample. The precise weight was noted and the above solution was added using a 10 ml disposable pipette, so that the polymer concentration was 2.5mg/ml. The tubes were covered with a sheet of Teflon, clamped and tumbled end-over-end (30-40 revolutions per minute) inside an atmospheric chamber at 37°C for three hours. The polymers were recovered by centrifugation at 500xg for 10 minutes and the supernatants were sampled, filtered through a 96 well 0.45 micron Whatman Unifilter 800 by centrifugation at 1000xg for 10 minutes to remove any remaining particulates. Filtrates were transferred to either glass IC vials with rubber septa or 96 well polypropylene deep well sample plates.

To determine the concentration of glycocholate (GC) and glycodeoxycholate (GDC) in the filtrate, 50 $\mu$L of the sample solution was injected onto a HPLC system, equipped with Phenomenex Luna C8 (2) column (100 Å, 5 $\mu$m, 50 x 2.00 mm,) and a UV detector. The sample was analyzed using a gradient of water, 25 mM phosphate buffer (pH=3) and acetonitrile at a flow rate of 0.4 mL/min. The signal of GC and GDC was detected at a wavelength of 205 nm from the UV detector. Calibration solutions comprised of GC and GDC standards of different concentrations were also injected onto the same HPLC system. The calibration curve of each component was then constructed by plotting the peak area vs. concentration. Based on the peak area of the GC and GDC found in the sample and the corresponding calibration curve, the concentration of each component in the sample was calculated in mM.

By comparing the equilibrium concentrations of glycocholate (GCeq) and glycodeoxycholate (GDCeq), in the presence of the polymer to their concentrations in test solution in the absence of the polymer, the amount of each component bound under these experimental conditions in mmoles/g polymer was calculated.

In some cases, the concentration of phosphate was also determined by injection of 20 ul of filtrate onto strong anion exchange columns (Dionex AG11-HC 50x4mm ID and Dionex AS11-HC 250x4mm ID) using a Waters Alliance 2795 Separation Module equipped with a 6 column switching valve installed inside a column oven and a Dionex Conductivity Detector CD25 (with DS3 flow cell and ASRS Ultra 11 4mm Suppressor). The mobile phase was 30 mM KOH buffer with a 1 ml/min flow rate and a run time of 15 minutes per sample. Phosphate standards of different concentrations were also injected onto the same system and the calibration curve was then constructed by plotting the peak area vs. concentration. Based on the peak area found in the sample and the corresponding calibration curve, the concentration of phosphate in the sample was calculated in mM.

By comparing the equilibrium concentrations of phosphate (Peq) and in the presence of the polymer to their concentrations in test solution in the absence of the polymer, the amount of phosphate bound under these experimental conditions in mmoles/g polymer was calculated.

Protocol 2: Conditions mimicking the upper small intestine (Assay B). Amine polymers were also measured in conditions mimicking those found in the upper small intestine after a meal (Fordtran, JS and Locklear, TW (1966) "Ionic constituents and osmolality of gastric and small-intestinal fluids after eating", Am J Dig Dis 11: 503-521; Northfield, TC

and McColl, I (1973) "Postprandial concentrations of free and conjugated bile salts down the length of the normal human small intestine", Gut 14: 513-518; Evans, DF, et al. (1988) "Measurement of gastrointestinal pH profiles in normal ambulant human subjects", Gut 29: 1035-1041). The bile salt binding performance of test polymers was evaluated at a polymer concentration of 2.5mg/ml in the manner described in Protocol 1 above, with the exception that the following test solution was used: 50mM N,N-Bis(2-hydroxyethyl)-2-aminoethanesulfonic acid (BES), 50 mM sodium BES, 6.5mM sodium phosphate, 4.6mM sodium glycocholate, 4.6mM sodium glycodeoxycholate, 1.2mM oleyl glycerol, 9mM oleic acid, 150mM sodium chloride, pH 7.0. Freeze-dried polymer was precisely dispensed into the 16x100mm borosilicate test tubes, with each tube containing between 28 and 33 mg of test sample. In certain cases, the concentration of polymer was adjusted from 2.5mg/ml to 1mg/ml. Otherwise the procedure was identical to that described in Protocol 1 above, except filtrates submitted for analytical analysis were only dispensed into glass IC vials.

To determine the concentration of glycocholate (GC), glycodeoxycholate (GDC), oleyl glycerol (OG) and oleic acid (OA) concentrations in filtrate samples, 20 $\mu$L was injected onto a HPLC system that was equipped with a Phenomenex Luna C8 (2) column (100 Å, 5 $\mu$m, 50 x 2.00 mm,) and a UV detector. The sample was analyzed using a gradient of water, 25 mM phosphate buffer (pH=3) and acetonitrile at a flow rate of 0.4 mL/min. The signal of GC, GDC, OG and OA is detected at a wavelength of 205 nm from the UV detector. Calibration solutions comprised of GC, GDC, OG and OA standards of different concentrations were also injected onto the same HPLC system. The calibration curve of each component was then constructed by plotting the peak area vs. concentration. Based on the peak area of the GC, GDC, OG or OA found in the sample and the corresponding calibration curve, the concentration of each component in the sample is calculated in mM.

By comparing the equilibrium concentrations of glycocholate (GCeq), glycodeoxycholate (GDCeq), oleyl glycerol (OGeq) and/or oleic acid (OAeq) in the presence of the polymer to their concentrations in test solution in the absence of the polymer, the amount of each component bound under these experimental conditions in mmoles/g polymer was calculated.

Hamster Model. To collect *in vivo* data, Male Golden Syrian hamsters (8-9 weeks old) were obtained from Charles River Laboratories (Wilmington, MA). Upon arrival, the animals were placed on rodent diet Teklad 2018 (Madison, WI). Food and water were provided ad libitum throughout the course of the study. Animals were acclimated for at least seven days, and then randomized by body weight into groups of at least five animals each. All animals were then placed on a high-fat, high-sucrose western diet, D12079B (Research Diet, New Brunswick, NJ) for three days before the study started. Amine polymers were blended into western diet at a dose of 0.5% to prepare the test diets. To initiate the study, all hamsters were moved into individual metabolic cages allowing the separation and collection of feces. Animals from the test groups were switched to the test diets, while animals from the untreated group were kept on western diet without added amine polymer. Food intake was measured for the next four consecutive days. For each hamster, feces from the last three days of the treatment period were collected, pooled, lyophilized, and then homogenized by grinding in a mortar and pestle. The feces samples were then extracted for fecal bile salt analysis.

In some cases, a baseline treatment period was conducted where all groups of animals were placed in metabolic cages as described above and fed only on western diet without added test article. Feces were collected as described above and the effect of amine polymer on bile salt fecal excretion was determined by comparing baseline versus treatment periods. Otherwise, the effect of amine polymer on bile salt fecal excretion was determined by comparing untreated versus test groups.

Hamster fecal bile salts were analyzed using a modification of the procedure reported by Porter and colleagues (Porter, JL. et al. 2003. Accurate enzymatic measurement of fecal bile salts in patients with malabsorption. J Lab Clin Med. 141:411-8). For each extraction, a 100mg aliquot of dry feces was weighed into a 16 x 100 mm Pyrex test tube. 1ml ethylene glycol with 0.7N NaOH was then added. The test tube was capped with a marble and heated at 190-200°C for 2 h. After cooling, 1ml of 20% NaCl and 0.2ml 6N HCl were added. After brief mixing, 6ml diethyl ether was added. The tube was capped, vortexed for 5 min, and then centrifuged at 1,000 x g for 5 min. The diethyl ether phase was transferred into a 20ml glass vial. Two additional extractions with 6ml diethyl ether were performed and the extracts were pooled. The ether was completely evaporated under a stream of air. The residue was then dissolved in 3ml methanol and bile salts (cholic acid, 3-OH-12Oxo-Cholanic Acid, chenodeoxycholic acid deoxycholic acid and lithocholic acid) were quantified by LC-MS.

<u>Example 1:</u> Aminopropyl imidazole (API) polymers with dibromooctane, dibromodecane and dibromododecane

Synthesis of imidazole crosslinked materials were conducted using parallel synthesis. An imidazole monomer was dispensed into 40 mL glass vials. A solution of the crosslinking monomer of formula X-$R_1$-X wherein X is bromo and $R_1$ is as listed in the table below (40 wt.% in N,N'-dimethyl formamide (DMF) in the case of 1,10-dibromodecane and 1,12-dibromododecane and neat in the case of dibromooctane), solvents and $Na_2CO_3$ were added to each vial. The vials were capped and heated for 17 hours at 70°C. Most vials contained a solid plug of polymer. The polymer was washed in water then swollen and ground in methanol, washed in methanol (twice), washed with aqueous hydrochloric

acid (0.5 M), water(once), sodium hydroxide (0.01 M, three times), water (two times) and lyophilized until dry.

| Sample # | Crosslinker ($R_1$) | Monomer: Crosslinking Monomer Molar Ratio | Crosslinking monomer | API | DMF | MeOH | $Na_2CO_3$ |
|---|---|---|---|---|---|---|---|
| | | | (mg) | (uL) | (uL) | (uL) | (mg) |
| 1-A3 | $C_8$ alkylene | 1:1.4 | 2258 | 707 | 1729 | 1729 | 1484 |
| 1-A4 | $C_{10}$ alkylene | 1:1.1 | 2175 | 786 | 1729 | 1729 | 1650 |
| 1-A5 | $C_{10}$ alkylene | 1:1.4 | 2311 | 657 | 1729 | 1729 | 1377 |
| 1-A6 | $C_{12}$ alkylene | 1:1.1 | 2228 | 736 | 1729 | 1729 | 1545 |
| 1-B1 | $C_{12}$ alkylene | 1:1.4 | 2358 | 612 | 1729 | 1729 | 1285 |

Bile acid binding capacity, affinity, and retention for each resulting polymer were determined via the A assay, B assay and hamster model and results are reported in the table below.

| Sample # | BA binding affinity | BA Binding capacity | BA Binding retention | BA binding | Swelling |
|---|---|---|---|---|---|
| | A assay (mmol/g) | B assay (mmol/g) | Hamster (mmol/g) | % 1° BA in feces* | (g/g) |
| 1-A3 | 0.48 | 2.68 | 0.54 | 3.5 | 9.59 |
| 1-A4 | 0.61 | 2.49 | 0.58 | 8.7 | 22.01 |
| 1-A5 | 0.61 | 2.34 | 0.61 | 11.2 | 10.49 |
| 1-A6 | 0.68 | 2.25 | | | 18.22 |
| 1-B1 | 0.68 | 2.09 | 0.58 | 21.7 | 4.67 |
| *%Primary (1°) Bile Acids in feces as % of total measured: i.e.<br>(Cholic acid + chenodeoxycholic acid) x 100/<br>(Cholic acid + chenodeoxycholic acid + 3-OH-12Oxo-Cholanic Acid + deoxycholic acid + lithocholic acid) | | | | | |

<u>Example 2:</u> Aminopropyl imidazole (API) polymers with dibromopropane - Comparator

Synthesis of imidazole crosslinked materials were conducted using parallel synthesis. An imidazole monomer was dispensed into a 40 mL glass vial. Dibromopropane and $Na_2CO_3$ were added to the vial. The vial was capped and heated for 17 hours at 70°C. The vial contained a solid plug of polymer. The polymer was washed in water then swollen and ground in methanol, washed in methanol (twice), washed with aqueous hydrochloric acid (0.5 M), water(once), sodium hydroxide (0.01 M, three times), water (two times) and lyophilized until dry.

| Sample # | Monomer: Crosslinking Monomer Molar Ratio | Dibromo-propane | API | DMF | MeOH | $Na_2CO_3$ |
|---|---|---|---|---|---|---|
| | | (mg) | (uL) | (uL) | (uL) | (mg) |
| 1-A1 | 1:1.4 | 1675 | 1263 | 1729 | 1729 | 2650 |

Bile acid binding capacity, affinity, and retention for each resulting polymer were determined via the A assay, B assay and hamster model and results are reported in the table below.

| Sample # | Bile acid binding affinity | Bile acid binding capacity | Bile acid binding Rentention | BA binding | Swelling |
|---|---|---|---|---|---|
| | A assay (mmol/g) | B assay (mmol/g) | Hamster (mmol/g) | %1° BA in feces* | (g/g) |
| 1-A1 | 0.57 | 2.29 | 0.40 | 14.8 | 32.20 |
| *%Primary Bile Acids in feces as % of total measured: i.e.<br>(Cholic acid + chenodeoxycholic acid) x 100/<br>(Cholic acid + chenodeoxycholic acid + 3-OH-12Oxo-Cholanic Acid + deoxycholic acid + lithocholic acid) | | | | | |

Example 3: Aminopropyl imidazole (API) polymers with dichloroxylene

Synthesis of imidazole crosslinked materials were conducted using dispensing robots with liquid and powder dispensing capabilities. An imidazole monomer was dispensed into 8 mL glass vials. Dichloroxylene, solvents and $Na_2CO_3$ were added to each vial in amounts shown in the table below. The vials were capped and heated for 17 hours at 70°C. Most vials contained a solid plug of polymer. The polymer was washed in water then swollen and ground in methanol, washed in methanol (twice), washed with aqueous hydrochloric acid (0.5 M), water(once), sodium hydroxide (0.01 M, three times), water (two times) and lyophilized until dry.

| Sample # | Monomer: Crosslinking Monomer Molar Ratio | Dichloroxylene | API | DMF | MeOH | $Na_2CO_3$ |
|---|---|---|---|---|---|---|
| | | (mg) | (uL) | (uL) | (uL) | (mg) |
| 2-B3 | 1:1.1 | 308 | 191 | 188 | 225 | 339 |
| 2-B4 | 1:1.4 | 392 | 191 | 219 | 262 | 339 |
| 2-B5 | 1:1.7 | 476 | 191 | 250 | 299 | 339 |
| 2-B6 | 1:2 | 559 | 191 | 282 | 336 | 339 |

Bile acid binding capacity, affinity, and retention for each resulting polymer were determined via the A assay, B assay and hamster model and results are reported in the table below.

| Sample # | Bile acid binding | Bile acid binding |
|---|---|---|
| | A assay (mmol/g) | B assay (mmol/g) |
| 2-B3 | 0.56 | 1.52 |
| 2-B4 | 0.54 | 1.13 |
| 2-B5 | 0.51 | 1.15 |
| 2-B6 | 0.54 | 1.34 |

Example 4: Aminopropyl imidazole (API) polymers with epichlorohydrin (ECH) - Comparator

Synthesis of imidazole crosslinked materials were conducted using dispensing robots with liquid and powder dispensing capabilities. An imidazole monomer was dispensed into a 40 mL glass vial. Epichlorohydrin (ECH) and water were added to the vial. The vials were capped and heated for 17 hours at 70°C. Most vials contained a solid plug of polymer. The polymer was swollen and ground in methanol, washed in methanol (twice), washed with aqueous hydrochloric acid (1 M), water (three times) and lyophilized until dry.

| Sample # | Monomer: Crosslinking Monomer Molar Ratio | ECH | API | Water |
|---|---|---|---|---|
| | | (uL) | (uL) | (uL) |
| 3-A1 | 1:1.1 | 910 | 1262 | 2400 |

Bile acid binding capacity, affinity, and retention for each resulting polymer were determined via the A assay, B assay and hamster model and results are reported in the table below.

| Sample # | Bile acid binding | Bile acid binding | Bile acid binding | BA binding | Swelling |
|---|---|---|---|---|---|
| | A assay (mmol/g) | B assay (mmol/g) | Hamster (mmol/g) | %Primary BA in feces* | (g/g) |
| 3-A1 | 0.35 | 2.80 | 0.22 | 6.7 | 13.82 |
| *%Primary Bile Acids in feces as % of total measured: i.e. (Cholic acid + chenodeoxycholic acid) x 100/ (Cholic acid + chenodeoxycholic acid + 3-OH-12Oxo-Cholanic Acid + deoxycholic acid + lithocholic acid) | | | | | |

Example 5: Aminopropyl imidazole (API) polymers with diepoxide crosslinkers

Synthesis of imidazole crosslinked materials were conducted using dispensing robots with liquid and powder dispensing capacities. An imidazole monomer was dispensed into 40 mL glass vials. The diepoxide crosslinking monomer and solvent, if used, were added to each vial in the amounts shown in the table below. The vials were capped and heated for 17 hours at 70°C. Most vials contained a solid plug of polymer. The polymer was swollen and ground in methanol, washed in methanol (twice), washed with aqueous hydrochloric acid (0.5 M for samples 2-A3 and A4, and 1 M for samples 4-B5 and B6), water (once), sodium hydroxide (0.01 M, three times - only in case of samples 2-A3 and A4), water (twice) and lyophilized until dry.

| Sample # | Monomer: Crosslinking Monomer Molar Ratio | Diepoxide crosslinking Monomer | Crosslinking monomer | API | DMF |
|---|---|---|---|---|---|
| | | | (uL) | (uL) | (uL) |
| 4-B5 | 1:1.7 | 1,3-Butadiene diepoxide | 291 | 264 | 636 |
| 4-B6 | 1:2 | 1,3-Butadiene diepoxide | 312 | 241 | 636 |
| 2-A3 | 1:1.1 | 1,2,7,8-Diepoxyoctane | 251 | 191 | |
| 2-A4 | 1:1.4 | 1,2,7,8-Diepoxyoctane | 319 | 191 | |

Bile acid binding capacity, affinity, and retention for each resulting polymer were determined via the A assay, B assay and hamster model and results are reported in the table below:

| Sample # | BA binding affinity | BA binding capacity |
|---|---|---|
| | A assay (mmol/g) | B assay (mmol/g) |
| 4-B5 | 0.32 | 1.31 |
| 4-B6 | 0.31 | 1.17 |
| 2-A3 | 0.46 | 1.49 |
| 2-A4 | 0.47 | 0.95 |

Example 6: Aminopropyl imidazole (API) polymers with trimethylolpropane triglycidyl ether (TTE)

Synthesis of imidazole crosslinked materials were conducted using dispensing robots with liquid and powder dispensing capabilities. An imidazole monomer was dispensed into 8 mL glass vials. Trimethylolpropane triglycidyl ether (TTE) and N,N'-dimethyl formamide (DMF) were added to each vial in the amounts shown in the table below. The vials were capped and heated for 17 hours at 70°C. Most vials contained a solid plug of polymer. The polymer was swollen and ground in methanol, washed in methanol (twice), washed with aqueous hydrochloric acid (1 M), water (three times) and lyophilized until dry.

| Sample # | Monomer: Crosslinking Monomer Molar Ratio | TTE | API | DMF |
|---|---|---|---|---|
| | | (uL) | (uL) | (uL) |
| 5-C4 | 1:0.81 | 343 | 193 | 636 |
| 5-C5 | 1:0.97 | 363 | 171 | 636 |
| 5-C6 | 1:1.13 | 379 | 153 | 636 |

Bile acid binding capacity, affinity, and retention for each resulting polymer were determined via the A assay, B assay and hamster model and results are reported in the table below.

| Sample # | BA binding affinity | BA binding capacity |
|---|---|---|
| | A assay (mmol/g) | B assay (mmol/g) |
| 5-C4 | 0.38 | 1.97 |
| 5-C5 | 0.40 | 1.73 |
| 5-C6 | 0.43 | 1.49 |

Example 9: 2-(2-Methylimidazolyl)ethylamine dihydrochloride (MIEA) with dibromodecane (DBD)

Synthesis of imidazole crosslinked materials were conducted using dispensing robots with liquid and powder dispensing capabilities. An imidazole monomer was dispensed into 8 mL glass vials. Dibromodecane (DBD) and solvents and sodium carbonate were added to each vial in the amounts shown in the table below. The vials were capped and heated for 17 hours at 70°C. Most vials contained a solid plug of polymer. The polymer was swollen and ground in methanol, washed in methanol (twice), washed with aqueous hydrochloric acid (0.5 M), water (three times) and lyophilized until dry.

| Sample # | Monomer: Crosslinking Monomer Molar Ratio | MIEA | DBD | NMP | Water | $Na_2CO_3$ |
|---|---|---|---|---|---|---|
| | | (mg) | (mg) | (uL) | (uL) | (mg) |
| 8-B3 | 1:1.1 | 113 | 187 | 235 | 78 | 120 |
| 8-B4 | 1:1.4 | 96 | 204 | 235 | 78 | 103 |
| 8-B5 | 1:1.7 | 84 | 216 | 235 | 78 | 90 |

Bile acid binding capacity, affinity, and retention for each resulting polymer were determined via the A assay, B assay and hamster model and results are reported in the table below.

| Sample # | BA binding affinity | BA binding capacity |
|---|---|---|
| | A assay (mmol/g) | B assay (mmol/g) |
| 8-B3 | 0.62 | 2.29 |
| 8-B4 | 0.64 | 1.86 |
| 8-B5 | 0.63 | 1.84 |

Example 18: Emulsion Polymerization of crosslinked imidazole functional monomer or amine functional monomer

Synthesis of imidazole crosslinked materials were conducted using parallel synthesis. An imidazole monomer (5.5g, 30.34mmole) listed in the table below was dispensed into 40 mL glass vials equipped with overhead stirrer, condenser, thermometer and an oil bath. Water was then added (13.1 mL) and the resulting mixture was stirred for 5 minutes. Aqueous surfactant dodecylbenzenesulfonic acid sodium salt (3.38g, 15wt.% aqueous solution) was then added. The resulting mixture stirred at 100 rpm for 10 minutes. A solution of the crosslinking monomer as listed in the table below (3.37g, 36.41 mmole) was added. The organic layer was then charged to the reactor; heptanes (26.18 mL)

followed by Sorbitan oleate (Span 80) solution (24.62 mL, 15 wt.% in heptanes). The final mixture was stirred at 200 rpm with an overhead stirrer. The internal temperature of the reaction was at 70°C. After 17 hours, Dean-Stark treatment at 80°C was performed to remove the water using the azeotrope of heptanes and water. This was achieved by increasing the temperature of the circulating oil bath to 100°C for 3 hours then increasing it further to 110°C. The process end point was identified by the temperature of the mixture reaching 98°C or until all the water initially added into the reaction mixture was collected.

The reaction mixture was allowed to cool to ambient temperature, stirring was stopped and the organic layer was decanted off. The resulted product was washed with heptanes twice, isopropyl alcohol twice, and finally with N-Methyl pyrrolidone once.

*Hydrophobic Modification of above beads.* The beads collected after the washing step from above (5.5 g) were dispersed in 28.17 mL N-methylpyrrolidone solvent. Solution of the hydrophobic monomer of formula X-$R_1$-X wherein X is bromo and $R_1$ of is as listed in the table below (9.96 g, 30.34 mmole) was then added and the mixture was stirred at 200 rpm, 80°C for 17 hours. The resulting product was washed with N-methylpyrrolidone once, methanol twice, 0.5M hydrochloric acid twice, water once, sodium bicarbonate twice, and finally water three times. The product was lyophilized to dryness for 24 hours.

| Sample # | Monomer | Crosslinker | Mon/ Crosslinker ratio | Post Hydrophobic modification | Monomer/ hydrophobic |
|---|---|---|---|---|---|
| 14-A1 | Mon 1 | ECH | 1:2 | Hyph 1 | 1:1 |
| 15-A1 | Mon 2 | DCP | 1:2 | Hyph 1 | 1:1 |
| 16-A1 | Mon 3 | Xlin 1 | 1:2 | Hyph 2 | 1:1 |

Abbreviation:

Mon 1: 7-(1H-imidazol-1-yl)heptan-1-amine
Mon2: 7-(2-methyl-H-imidazol-1-yl)heptan-1-amine
Mon3: bis-(3-aminopropyl)amine
ECH: epichlorohydrin
DCP: 1,3-dichloropropanol
Xlin 1: 4,4'-(propane-1,3-diyl)bis(1-(10-bromodecyl)-1-methylpiperidinium)
Hyph1: 1,12-dibromododecane
Hyph2: 1,10-dibromodecane

Bile acid binding capacity, affinity, and retention for each resulting polymer were determined via the A assay, B assay and hamster model and results are reported in the table below.

| Sample # | Bile acid binding capacity B assay (mmol/g) | Bile acid binding affinity A assay (mmol/g) | Bile acid binding retention Hamster (mmol/g) | Swelling (g/g) |
|---|---|---|---|---|
| 14-A1 | 2.09 | 0.55 | 0.19 | 2.48 |
| 15-A1 | 2.79 | 0.41 | | |
| 16-A1 | 2.53 | 0.62 | | 20.5 |

Example 19: Solution Polymerization of crosslinked imidazole or amine or epoxy functional monomer

Synthesis of imidazole crosslinked materials were conducted using parallel synthesis. An imidazole monomer listed in the table below was dispensed into 40 mL glass vials equipped with magnetic stirrer. Solvent was then added; water was used for water soluble crosslinker and N-Methylpyrrolidone for organic soluble crosslinkers. The resulting solution was stirred for 5 minutes. A solution of the crosslinking monomer as listed in the table below was then added. Vial was capped and heated at 70°C with stirring for 17 hours. A clear hard gel was obtained. The gel was ground with an ultrasonic mixer for 30 minutes in methanol and then washed with methanol twice, 0.5 M hydrochloric acid once, water once, sodium bicarbonate twice, and finally water three times.

| Sample # | Monomer | Crosslinker | Monomer/Crosslinker ratio |
|---|---|---|---|
| 17-A1 | Mon5 | Xlin 2 | 1:5 |
| 18-A1 | Mon6 | Xlin 2 | 1:7 |
| 18-A2 | Mon7 | Xlin 2 | 1:7 |
| 19-A2 | mon 1 | ECH | 1:4 |
| 20-A1 | Mon8 | ECH | 1:4 |
| 21-A1 | Mon9 | ECH | 1:4 |
| 22-A1 | Mon9 | Xlin 3 | 1:2 |
| 23-A1 | Mon6 | Xlin 2 | 1:1 |

Abbreviation:

Mon 1: 7-(1H-imidazol-1-yl)heptan-1-amine
Mon5: 4-decyldipropyl triamine
Mon6: 1,10-diaminodecane
Mon7: 1,12-diaminododecane
Mon8: 3-(12-aminododecyl)-1-(3-aminopropyl)-2-methyl-1H-imidazol-3-ium Bromide
Mon9: 1,3-bis(7-aminoheptyl)-1H-imidazol-3-ium bromide
Xlin2: 2-methyl-1,3-bis(oxiran-2-ylmethyl)-1H-imidazol-3-ium
ECH: epichlorohydrin
Xlin3: 1,12-dibromododecane

Bile acid binding capacity, affinity, and retention for each resulting polymer were determined via the A assay, B assay and hamster model and results are reported in the table below.

| Sample # | Crosslinker: Monomer Ratio | ECH | API | Water | heptane | DBSA | Span 80 |
|---|---|---|---|---|---|---|---|
| | | (mL) | (mL ) | (mL) | (mL) | (mL, in water, 15 wt.%) | (mL, in heptanes, 15 wt.%) |
| 24-A1 | 1:1.4 | 17.4 | 19.1 | 59.2 | 117.8 | 14.8 | 110.8 |
| 25-C2 | 1:1.1 | 13.7 | 19.1 | 46.6 | 105.9 | 20.7 | 99.6 |

Example 25: Synthesis of 4,4'-(propane-1,3-diyl)bis(1-(11-bromoundecyl)-1-methylpiperidinium)

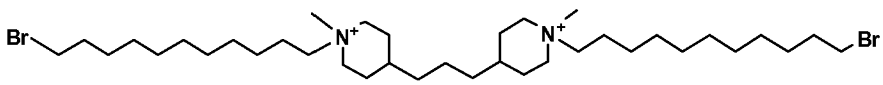

A mixture of 11-bromo-undecanol (31.65g, 0.126mol) and 4,4'-trimethylenebis(1-methylpiperidine) (5 g, 0.021 mol) in methanol (50 mL) was refluxed for 17 hours. Methanol was removed by rotary evaporation. To the residue was added toluene (100 mL) and the mixture was stirred at 50°C for 2hours. The solvent was removed by filtration. The solid was washed with toluene (100 mL) and ether (2x100mL). After drying under high vacuum, 4,4'-(propane-1,3-diyl)bis(1-(11-hydroxyundecyl)-1-methylpiperidinium) was obtained as a white powder (15.5g, 100% yield). MS m/e (M2+), calculated 290.3, found 290.5.

4,4'-(Propane-1,3-diyl)bis(1-(11-hydroxyundecyl)-1-methylpiperidinium) (15.5 g, 0.21 mol) was placed in a pressure flask. Hydrobromic acid (48% in water, 50 mL) was added in and the flask was tightened to seal. The reaction was stirred at 120°C for 17 hours. The reaction mixture was azeotroped with THF and toluene to removed excess hydrobromic

acid. The residue was dried in vacuum oven at 35°C for 24 hours to give 17.27 g crude product as a light brown powder (94.9%).

The crude product (4.02g) was recrystallized in isopropanol (20 mL) to give 4,4'-(propane-1,3-diyl)bis(1-(11-bromoundecyl)-1-methylpiperidinium) as an off-white solid (3.11 g, 77.4% recovery). MS m/e (M2+), calculated 353.2, found 353.3.

Example 26: Synthesis of 4,4'-(propane-1,3-diyl)bis(1-(10-bromodecyl)-1-methylpiperidinium)

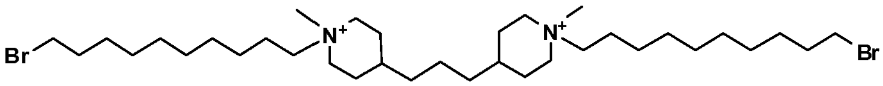

Dibromodecane (94.5 g, 0.32 moles) and 40 mL of methanol were added to a round bottom flask. The flask was heated to 55°C for 15 minutes. The mixture became a clear, yellow solution. 4,4'-Trimethylenebis(1-methylpiperidine) (15g, 0.063 mol) was added to the solution and the reaction mixture was allowed to stir at 55°C for 17 hours. After cooling to the room temperature, the reaction mixture was poured into 450 mL of 3:1 acetone:hexane. Solvent was removed after centrifugation. The solid was washed with hexane (500 mL) and diethyl ether (500 mL). After drying in a vacuum oven at 35°C for 17 hours, 47.43 g of product was obtained as a pale yellow solid (89.8% yield). MS m/e (M2+), calculated 339.4, found 339.2.

Example 27: General procedure for hydrogenation

The nitrile substrate, Raney-cobalt (40 wt.%) and water (60 mL) were charged into the reaction vessel, which was put under vacuum and then filled with hydrogen (700 psi). The reaction mixture was heated at 100°C and stirred about 30 minutes until the pressure stabilized. After being cooled to room temperature, the reaction mixture was filtered through a pad of Celite and washed with water. The filtrate was concentrated to give the product.

4,4'-(Propane-1,3-diyl)bis(1-(6-aminohexyl)-1-methylpiperidinium) was synthesized from the nitrile precursor according to the general synthesis listed above.

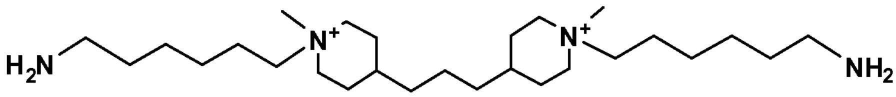

Example 33: Synthesis of 7-(1H-imidazol-1-yl) heptan-1-amine

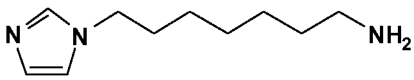

7-(1H-Imidazol-1-yl)heptanenitrile (12.86 g, 0.072 mol) was dissolved in toluene (60 mL). The hydrogenation procedure described above was followed to produce 7-(1H-imidazol-1-yl)heptan-1-amine as a yellow oil (12.50 g, 95% yield). MS m/e (MH+), calculated 182.17, found 181.98. $^1$HNMR confirmed structure.

Example 34: Synthesis of 7-(2-methyl-1H-imidazol-1-yl)heptan-1-amine

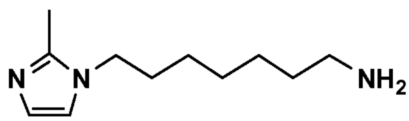

1-(2-Cyanoethyl)2-methylimidazole (17.8 g, 0.13mol) was dissolved in acetonitrile (100 mL). To the solution

was added 7-bromoheptanenitrile (25 g, 0.13 mol). The reaction was stirred at 90°C overnight. The solvent was removed under reduced pressure. To the residue was added chloroform (100 mL), followed by 20 wt.% sodium hydroxide solution (50 mL). The mixture was stirred at room temperature for 1 hour. The organic phase was separated and the aqueous phase was extracted with chloroform (50 mL). The combined organic phases were washed with brine (3x100 mL), water (2x100 mL) and dried over sodium sulfate. The solvent was removed under reduced pressure. 7-(2-Methyl-1H-imidazol-1-yl)heptanenitrile was obtained as a yellow oil in quantitative yield. MS m/e (MH+), calculated 192.14, found 192.5. $^1$HNMR conformed structure.

7-(2-Methyl-1H-imidazol-1-yl)heptanenitrile (25g) was dissolved in toluene (60 mL) and following the hydrogenation procedure described above, 7-(2-methyl-1H-imidazol-1-yl)heptan-1-amine was obtained as a brown oil (15 g, 59% yield). MS m/e (MH+), calculated 196.17, found 196.20. 1HNMR confirmed structure.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above polymers, pharmaceutical compositions, and methods of treatment without departing from the scope of the invention, it is intended that all matter contained in the above description and shown in the accompanying drawing[s] shall be interpreted as illustrative and not in a limiting sense.

## Claims

1. An amine polymer comprising repeat units derived from polymerization of a crosslinking monomer and an imidazole monomer of formula 1 or a salt thereof

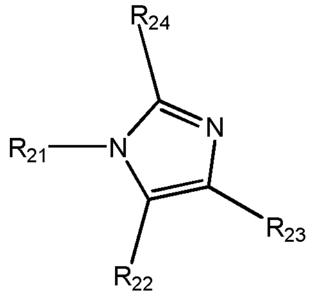

(1)

wherein

$R_{21}$, $R_{22}$, $R_{23}$, and $R_{24}$ are independently hydrogen, $C_1$ to $C_{12}$ alkyl, aryl, or heterocyclo;
provided that at least one of $R_{21}$, $R_{22}$, $R_{23}$, and $R_{24}$ is -$R_2$-NH-$R_{26}$ and the other R groups are less reactive with the crosslinking monomer than the -$R_2$-NH-$R_{26}$ nitrogen or the imidazole nitrogens;
$R_2$ is $C_2$ to $C_{14}$ alkylene;
$R_{26}$ is hydrogen, $C_1$ to $C_{16}$ hydrocarbyl, $C_1$ to $C_{16}$ substituted hydrocarbyl, or $C_1$ to $C_{50}$ alkyl wherein the -$CH_3$ group or one or more of the -$CH_2$- groups are replaced with an amide, a carbonyl, an ether, an ester, a cycloalkyl, an aryl, or a heterocyclo functional group, or $C_1$ to $C_{50}$ alkyl wherein the -$CH_3$ group or one or more of the -$CH_2$- groups are substituted with a hydroxy, a halo, an amino, an alkoxy, or an aryloxy; and
the polymer segment derived from the crosslinking monomer has a calculated logP (cLog P) greater than 0.1.

2. The amine polymer of claim 1 wherein the imidazole monomer has the structure of formula 2

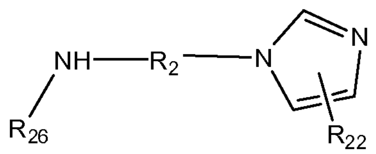

(2)

wherein

$R_{22}$ is hydrogen, $C_1$ to $C_{12}$ alkyl, aryl, or heterocyclo;
$R_2$ is $C_2$ to $C_{14}$ alkylene;
$R_{26}$ is hydrogen, $C_1$ to $C_{20}$ alkyl, or $C_1$ to $C_{20}$ substituted alkyl.

**3.** The amine polymer of claim 2 wherein the imidazole monomer has the structure of formula 2A

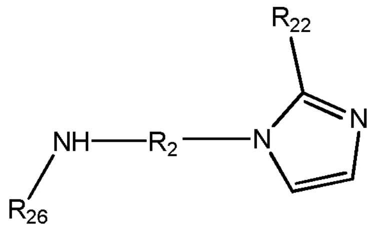

(2A)

wherein
$R_{22}$ is hydrogen or $C_1$ to $C_{12}$ alkyl.

**4.** The amine polymer of claim 3 wherein $R_{22}$ is methyl.

**5.** The amine polymer of claim 2 or 3 wherein $R_{22}$ is hydrogen, methyl, ethyl, or propyl; $R_2$ is $C_3$ to $C_6$ alkylene; and $R_{26}$ is hydrogen or $C_1$ to $C_6$ amino-substituted alkyl.

**6.** The amine polymer of claim 2 or 3 wherein $R_{22}$ is hydrogen, $R_2$ is propylene, and $R_{26}$ is hydrogen.

**7.** The amine polymer of any one claims 1 to 6 wherein the crosslinking monomer has 2 to 4 possible reactive sites and is susceptible to nucleophilic substitution.

**8.** The amine polymer of any one of claims 1 to 7 wherein the crosslinking monomer is 1,2-dibromoethane, 1,3-dichloropropane, 1,2-dichloroethane, 1-bromo-2-chloroethane, 1,3-dichloropropane, 1,3-dibromopropane, 1,4-dichlorobutane), 1,4-dibromobutane, 1,5-dichloropentane, 1,5-dibromopentane, 1,6-dichlorohexane, 1,6-dibromohexane, 1,7-dichloroheptane, 1,7-dibromoheptane, 1,8-dichlorooctane, 1,8-dibromooctane, 1,9-dichlorononane, 1,9-dibromononane, 1,10-dichlorodecane, 1,10-dibromodecane, 1,11-dibromoundecane, 1,11-dichloroundecane, 1,12-dichlorododecane, 1,12-dibromododecane, or a combination thereof.

**9.** The amine polymer of claim 8 wherein the crosslinking monomer is 1,8-dichlorooctane, 1,8-dibromooctane, 1,9-dichlorononane, 1,9-dibromononane, 1,10-dichlorodecane, 1,10-dibromodecane, 1,12-dichlorododecane, 1,11-dibromoundecane, 1,11-dichloroundecane, 1,12-dibromododecane, or a combination thereof.

**10.** The amine polymer of any one of claims 1 to 9 wherein the molar ratio of the imidazole monomer to the crosslinking monomer is from about 1:1 to about 1:5 when the crosslinking monomer is difunctional.

**11.** The amine polymer of any one of claims 1 to 9 wherein the molar ratio of the imidazole monomer to the crosslinking monomer is from about 2:1 to about 1:5 when the crosslinking monomer is trifunctional.

**12.** The amine polymer of any one of claims 1 to 11 wherein the amine polymer is for administering to an animal subject.

**13.** A pharmaceutical composition comprising a pharmaceutically acceptable excipient and an amine polymer of any one of claims 1 to 12.

**14.** An amine polymer of any one of claims 1 to 12 or a pharmaceutical composition of claim 13 for reducing serum LDL-cholesterol in an animal subject.

## Patentansprüche

**1.** Ein Aminpolymer, das sich wiederholende Einheiten enthält, die aus der Polymerisation eines vernetzenden Monomers und eines Imidazol-Monomers der Formel 1 oder eines Salzes davon abgeleitet sind,

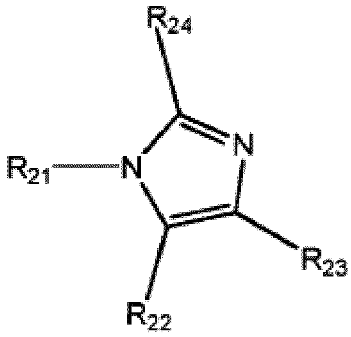

(1)

wobei

$R_{21}$, $R_{22}$, $R_{23}$, und $R_{24}$ unabhängig Wasserstoff, $C_1$ bis $C_{12}$ Alkyl, Aryl oder Heterocyclo sind;
unter der Voraussetzung, dass wenigstes eines von $R_{21}$, $R_{22}$, $R_{23}$ und $R_{24}$ $-R_2-NH-R_{26}$ ist und die anderen Gruppen R mit dem vernetzenden Monomer weniger reaktiv sind als der Stickstoff in $-R_2-NH-R_{26}$ oder als die Imidazol-Stickstoffe;
$R_2$ $C_2$ bis $C_{14}$ Alkylen ist;
$R_{26}$ Wasserstoff, $C_1$ bis $C_{16}$ Hydrocarbyl, $C_1$ bis $C_{16}$ substituiertes Hydrocarbyl, oder $C_1$ bis $C_{50}$ Alkyl ist, wobei die Gruppe $-CH_3$ oder eine oder mehrere der Gruppen $-CH_2-$durch ein Amid, ein Carbonyl, einen Ether, einen Ester, ein Cycloalkyl, ein Aryl, oder eine heterocyclische funktionelle Gruppe oder $C_1$ bis $C_{50}$ Alkyl ersetzt ist, wobei die Gruppe $-CH_3$ oder eine oder mehrere der Gruppen $-CH_2-$ mit einem Hydroxy, einem Halogen, einem Amin, einem Alkoxy oder einem Aryloxy substituiert sind; und
das aus dem vernetzenden Monomer abgeleitete Segment des Polymers einen berechneten logP (cLog P) größer als 0,1 aufweist.

**2.** Aminpolymer aus Anspruch 1, wobei das Imidazol-Monomer eine Struktur der Formel 2 aufweist

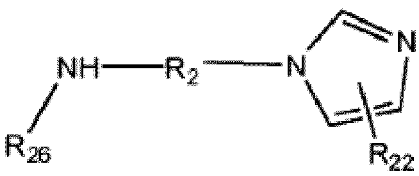

(2)

wobei

$R_{22}$ Wasserstoff, $C_1$ bis $C_{12}$ Alkyl, Aryl oder Heterocyclo ist;
$R_2$ $C_2$ bis $C_{14}$ Alkylen ist;
$R_{26}$ Wasserstoff, $C_1$ bis $C_{20}$ Alkyl oder $C_1$ bis $C_{20}$ substituiertes Alkyl ist.

**3.** Aminpolymer aus Anspruch 2, wobei das Imidazol-Monomer eine Struktur der Formel 2A aufweist

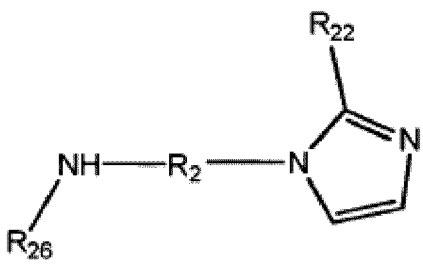

(2A)

wobei
$R_{22}$ Wasserstoff oder $C_1$ bis $C_{12}$ Alkyl ist.

**4.** Aminpolymer aus Anspruch 3, wobei $R_{22}$ Methyl ist.

**5.** Aminpolymer aus Anspruch 2 oder 3, wobei $R_{22}$ Wasserstoff, Methyl, Ethyl, oder Propyl ist; $R_2$ $C_3$ bis $C_6$ Alkylen ist; und $R_{26}$ Wasserstoff oder $C_1$ bis $C_6$ Aminosubstituiertes Alkyl ist.

**6.** Aminpolymer aus Anspruch 2 oder 3, wobei $R_{22}$ Wasserstoff ist, $R_2$ Propylen ist, und $R_{26}$ Wasserstoff ist.

**7.** Aminpolymer aus einem der Ansprüche 1 bis 6, wobei das vernetzende Monomer 2 bis 4 mögliche reaktive Stellen aufweist und für nukleophile Substitution empfänglich ist.

**8.** Aminpolymer aus einem der Ansprüche 1 bis 7, wobei das vernetzende Monomer 1,2-Dibromethan, 1,3-Dichlorpropan, 1,2-Dichlorethan, 1-Brom-2-chlorethan, 1,3-Dichlorpropan, 1,3-Dibrompropan, 1,4-Dichlorbutan, 1,4-Dibrombutan, 1,5-Dichlorpentan, 1,5-Dibrompentan, 1,6-Dichlorhexan, 1,6-Dibromhexan, 1,7-Dichlorheptan, 1,7-Dibromheptan, 1,8-Dichloroctan, 1,8-Dibromoctan, 1,9-Dichlornonan, 1,9-Dibromnonan, 1,10-Dichlordecan, 1,10-Dibromdecan, 1,11-Dibromundecan, 1,11-Dichlorundecan, 1,12-Dichlordodecan, 1,12-Dibromdodecan oder eine Kombination davon ist.

**9.** Aminpolymer aus Anspruch 8, wobei das vernetzende Monomer 1,8-Dichloroctan, 1,8-Dibromoctan, 1,9-Dichlornonan, 1,9-Dibromnonan, 1,10-Dichlordecan, 1,10-Dibromdecan, 1,12-Dichlordodecan, 1,11-Dibromundecan, 1,11-Dichlorundecan, 1,12-Dibromdodecan oder eine Kombination davon ist.

**10.** Aminpolymer aus einem der Ansprüche 1 bis 9, wobei das molare Verhältnis des Imidazol-Monomers zum vernetzenden Monomer von etwa 1:1 bis etwa 1:5 beträgt, wenn das vernetzende Monomer difunktionell ist.

**11.** Aminpolymer aus einem der Ansprüche 1 bis 9, wobei das molare Verhältnis des Imidazol-Monomers zum vernetzenden Monomer von etwa 2:1 bis etwa 1:5 beträgt, wenn das vernetzende Monomer trifunktionell ist.

**12.** Aminpolymer aus einem der Ansprüche 1 bis 11, wobei das Aminpolymer zur Verabreichung an ein tierisches Subjekt bestimmt ist.

**13.** Eine pharmazeutische Zusammensetzung, enthaltend einen pharmazeutisch akzeptablen Arzneistoffträger und ein Aminpolymer aus einem der Ansprüche 1 bis 12.

**14.** Ein Aminpolymer aus einem der Ansprüche 1 bis 12 oder eine pharmazeutische Zusammensetzung des Anspruchs 13 zur Verminderung des LDL-Cholesterins im Serum in einem tierischen Subjekt.

## Revendications

**1.** Polymère d'amine comprenant des motifs répétés résultant de la polymérisation d'un monomère de réticulation et d'un monomère imidazole de formule 1 ou d'un sel de celui-ci

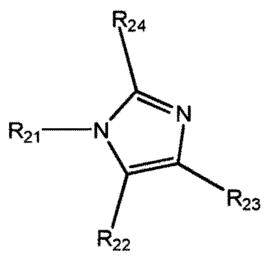

(1)

dans laquelle

$R_{21}$, $R_{22}$, $R_{23}$ et $R_{24}$ représentent indépendamment un atome d'hydrogène, un groupe aryle, hétérocyclo ou alkyle en $C_1$ à $C_{12}$ ;
sous réserve qu'au moins l'un des $R_{21}$, $R_{22}$, $R_{23}$ et $R_{24}$ représente $-R_2-NH-R_{26}$ et que les autres groupes R soient moins réactifs à l'égard du monomère de réticulation que l'azote $-R_2-NH-R_{26}$ ou les azotes d'imidazole ;

$R_2$ représente un groupe alkylène en $C_2$ à $C_{14}$;
$R_{26}$ représente un atome d'hydrogène, un groupe hydrocarbyle en $C_1$ à $C_{16}$, un groupe hydrocarbyle substitué en $C_1$ à $C_{16}$, ou un groupe alkyle en $C_1$ à $C_{50}$ dans lequel le groupe $-CH_3$ ou au moins l'un des groupes $-CH_2-$ est remplacé par un groupe fonctionnel amide, carbonyle, éther, ester, cycloalkyle, aryle ou hétérocyclo, ou un groupe alkyle en $C_1$ à $C_{50}$ dans lequel le groupe $-CH_3$ ou au moins l'un des groupes $-CH_2-$ est substitué par un groupe hydroxy, halo, amino, alcoxy ou aryloxy ; et
le segment polymère issu du monomère de réticulation présente un logP calculé (cLogP) supérieur à 0,1.

2. Polymère d'amine selon la revendication 1, dans lequel le monomère imidazole présente la structure de la formule 2

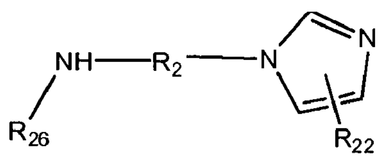

(2)

dans laquelle

$R_{22}$ représente un atome d'hydrogène, un groupe aryle, hétérocyclo ou alkyle en $C_1$ à $C_{12}$ ;
$R_2$ représente un groupe alkylène en $C_2$ à $C_{14}$ ;
$R_{26}$ représente un atome d'hydrogène, un groupe alkyle en $C_1$ à $C_{20}$ ou alkyle substitué en $C_1$ à $C_{20}$.

3. Polymère d'amine selon la revendication 2, dans lequel le monomère imidazole présente la structure de la formule 2A

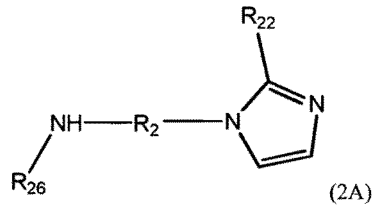

(2A)

dans laquelle
$R_{22}$ représente un atome d'hydrogène ou un groupe alkyle en $C_1$ à $C_{12}$.

4. Polymère d'amine selon la revendication 3 dans lequel $R_{22}$ représente un groupe méthyle.

5. Polymère d'amine selon la revendication 2 ou 3, dans lequel $R_{22}$ représente un atome d'hydrogène, un groupe méthyle, éthyle ou propyle ; $R_2$ représente un groupe alkylène en $C_3$ à $C_6$ ; et $R_{26}$ représente un atome d'hydrogène ou un groupe alkyle amino-substitué en $C_1$ à $C_6$.

6. Polymère d'amine selon la revendication 2 ou 3, dans laquelle $R_{22}$ représente un atome d'hydrogène, $R_2$ un groupe propylène et $R_{26}$ un atome d'hydrogène.

7. Polymère d'amine selon l'une quelconque des revendications 1 à 6, dans lequel le monomère de réticulation comporte de 2 à 4 sites réactifs possibles et est susceptible de faire l'objet d'une substitution nucléophile.

8. Polymère d'amine selon l'une quelconque des revendications 1 à 7, dans lequel le monomère de réticulation est le 1,2-dibromoéthane, le 1,3-dichloropropane, le 1,2-dichloroéthane, le 1-bromo-2-chloroéthane, le 1,3-dichloropropane, le 1,3-dibromopropane, le 1,4-dichlorobutane, le 1,4-dibromobutane, le 1,5-dichloropentane, le 1,5-dibromopentane, le 1,6-dichlorohexane, le 1,6-dibromohexane, le 1,7-dichloroheptane, le 1,7-dibromoheptane, le 1,8-dichlorooctane, le 1,8-dibromooctane, le 1,9-dichlorononane, le 1,9-dibromononane, le 1,10-dichlorodécane, le 1,10-dibromodécane, le 1,11-dibromoundécane, le 1,11-dichlorundécane, le 1,12-dichlorododécane, le 1,12-dibromododécane, ou une combinaison de ceux-ci.

9. Polymère d'amine selon la revendication 8, dans lequel le monomère de réticulation est le 1,8-dichlorooctane, le 1,8-dibromooctane, le 1,9-dichlorononane, le 1,9-dibromononane, le 1,10-dichlorodécane, le 1,10-dibromodécane,

le 1,12-dichlorododécane, le 1,11-dibromoundécane, le 1,11-dichlorundécane, le 1,12-dibromododécane, ou une combinaison de ceux-ci.

**10.** Polymère d'amine selon l'une quelconque des revendications 1 à 9, dans lequel le rapport molaire entre le monomère imidazole et le monomère de réticulation varie d'environ 1/1 à environ 1/5 lorsque le monomère de réticulation est difonctionnel.

**11.** Polymère d'amine selon l'une quelconque des revendications 1 à 9, dans lequel le rapport molaire entre le monomère imidazole et le monomère de réticulation varie d'environ 2/1 à environ 1/5 lorsque le monomère de réticulation est trifonctionnel.

**12.** Polymère d'amine selon l'une quelconque des revendications 1 à 11, dans lequel le polymère d'amine est destiné à être administré à un sujet animal.

**13.** Composition pharmaceutique comprenant un excipient de qualité pharmaceutique et un polymère d'amine selon l'une quelconque des revendications 1 à 12.

**14.** Polymère d'amine selon l'une quelconque des revendications 1 à 12 ou composition pharmaceutique selon la revendication 13, destinés à faire baisser le taux de cholestérol LDL sérique chez un sujet animal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5414068 A
- US 5727115 A
- US 5846966 A


### Non-patent literature cited in the description


- **FLORY P. J.** Principles of Polymer Chemistry. Cornell Ithica Pub, 1953
- **NORTHFIELD, TC ; MCCOLL, I.** Postprandial concentrations of free and conjugated bile salts down the length of the normal human small intestine. *Gut,* 1973, vol. 14, 513-518
- **BORGSTROM, B et al.** Studies of intestinal digestion and absorption in the human. *J Clin Invest,* 1957, vol. 36, 1521-1536
- **FORDTRAN, JS ; LOCKLEAR, TW.** Ionic constituents and osmolality of gastric and small-intestinal fluids after eating. *Am J Dig Dis,* 1966, vol. 11, 503-521
- **EVANS, DF et al.** Measurement of gastrointestinal pH profiles in normal ambulant human subjects. *Gut,* 1988, vol. 29, 1035-1041
- **PORTER, JL. et al.** Accurate enzymatic measurement of fecal bile salts in patients with malabsorption. *J Lab Clin Med,* 2003, vol. 141, 411-8